# EUROPEAN PATENT SPECIFICATION

(11) **EP 4 575 727 B1**
(45) Date of publication and mention of the grant of the patent: **21.01.2026**
(21) Application number: 24220163.0
(22) Date of filing: 16.12.2024
(51) Int. Cl.: G06F 3/01, G05B 19/18, G05B 23/02, G05B 19/418, G06V 10/774, G06V 10/82, G06V 20/20

(54) **METHOD AND APPARATUS FOR FEEDBACKING HUMAN-MACHINE COLLABORATION STATE BASED ON VIRTUAL-REAL INTEGRATION, AND ELECTRONIC DEVICE**
VERFAHREN UND VORRICHTUNG ZUR RÜCKMELDUNG DES MENSCH-MASCHINE-KOLLABORATIONSSTATUS AUF BASIS VON VIRTUELLER-REELLER INTEGRATION, UND ELEKTRONISCHE VORRICHTUNG
PROCÉDÉ ET APPAREIL DE RÉTROACTION D'ÉTAT DE COLLABORATION HOMME-MACHINE SUR LA BASE D'UNE INTÉGRATION VIRTUELLE-RÉELLE, ET DISPOSITIF ÉLECTRONIQUE

(30) Priority: 22.12.2023 CN 202311790016
(43) Date of publication of application: 25.06.2025
(73) Proprietor: Kingfar International Inc., Beijing 100080 (CN)
(72) Inventor: ZHAO, Qichao, Beijing, 100080 (CN); WANG, Qingju, Beijing, 100080 (CN)
(74) Representative: Range, Christopher William

(56) References cited:
- EP-A1- 3 479 971
- CN-A- 115 984 829
- US-A1- 2021 170 590

## Description

### FIELD

The present disclosure relates to the field of human-machine collaboration technologies, and more particularly, to a method and an apparatus for feedbacking a human-machine collaboration state based on virtual-real integration, and an electronic device.

### BACKGROUND

With the development of industrialization, automation, and intelligence, artificial intelligence, virtual reality technology, and augmented reality technology are widely applied in a human-machine collaboration system to promote interaction and collaboration between humans and machines. Therefore, there is a need to provide a method for feedbacking a human-machine collaboration state based on virtual-real integration.

For example, EP3479971A1 discloses a method of performing an assembling of an object. The method includes: monitoring, by an assembly system using a camera, a work space wherein each operator performed step is being performed; identifying, by a controller, at least one of the operator performed steps as an assembling step that precedes at least one of the machine performed steps; and controlling a robotic manipulation tool such as to synchronize performing of at least one machine performed step dependent on a state of completion of the identified operator performed step.

For example, US 2021170590A1 discloses a process control method for detecting an anomaly in an execution of a task in mixed human-robot processes. The method includes: acquiring human-worker (HW) signals and robot operational signals from sensors; extracting from the HW signals, a task completion time, measurements relating to a state of the HW and a next sequenced task, and input into a Human Performance (HP) model to obtain a state of the HW, which is then inputted into a Human-Robot Interaction (HRI) model to determine a classification of an anomaly or no anomaly; updating the HRI model robot and determine a control action of a robot or a type of an anomaly alarm using the updated HRI model and the classified anomaly; and outputting the control action of the robot to change a robot action or output the type of the anomaly alarm to a management system of the mixed human-robot processes.

### SUMMARY

The present disclosure aims to solve one of the technical problems in the related art at least to some extent. To this end, the present disclosure provides a method and an apparatus for feedbacking a human-machine collaboration state based on virtual-real integration, and an electronic device. A method for feedbacking a human-machine collaboration state based on virtual-real integration as recited in amended claims 1-10 is provided, an apparatus for feedbacking a human-machine collaboration state based on virtual-real integration as recited in amended claim 11 is provided, and a computer-readable storage medium as recited in amended claim 12 is provided.

The present disclosure provides a method for feedbacking a human-machine collaboration state based on virtual-real integration. The method includes: acquiring ergonomic data of an operation subject in a human-machine collaboration process for a current operation task and an operation scene image obtained by shooting the human-machine collaboration process, the operation scene image at least including an operation device and a setting parameter of an operation environment; performing a human-machine collaboration state recognition based on the operation scene image to obtain target collaboration state data corresponding to the current operation task; performing a personnel state recognition based on the ergonomic data to obtain personnel state data of the operation subject; and feedbacking a state of the human-machine collaboration process based on the target collaboration state data and the personnel state data.

In the present disclosure, firstly, the ergonomic data of the operation subject in the human-machine collaboration process for the current operation task, and the operation scene image that is obtained by shooting the human-machine collaboration process and at least includes the operation device and the setting parameter of the operation environment are acquired. Then, the human-machine collaboration state recognition is performed based on the operation scene image to obtain the target collaboration state data corresponding to the current operation task, and the personnel state recognition is performed based on the ergonomic data to obtain the personnel state data of the operation subject, so as to provide important information for the human-machine collaboration process. Finally, based on the target collaboration state data and the personnel state data, the state of the human-machine collaboration process is feedbacked to improve efficiency and safety of the human-machine collaboration process. A human-machine collaboration system makes corresponding adjustments based on the feedbacked different states to realize a more flexible and adaptive collaboration process. In addition, by feedbacking the state of the human-machine collaboration process, decisions can also be made for subsequent task execution. By optimizing procedures in the human-machine collaboration process and improving operating conditions and states of the operation subject, sustainable human-machine collaboration can be achieved.

The ergonomic data includes eye movement signal data, and the personnel state data includes task prediction state data. The performing the personnel state recognition based on the ergonomic data to obtain the personnel state data of the operation subject includes: performing a gaze position recognition based on the eye movement signal data to obtain a gaze position change of the operation subject, the gaze position change being used to describe a change of a gaze position of the operation subject with an execution time of the current operation task; and determining whether the operation subject predicts a next operation task of the current operation task based on the gaze position change to obtain the task prediction state data; wherein the current operation task corresponds to a current task region range, and the next operation task corresponds to a next task region range; and said determining whether the operation subject predicts the next operation task of the current operation task based on the gaze position change to obtain the task prediction state data comprises: determining whether at least part of gaze resources of the operation subject are transferred from the current task region range to the next task region range based on the gaze position change to obtain the task prediction state data.

In this embodiment, the gaze position recognition is performed based on the eye movement signal data to obtain the gaze position change of the operation subject. Whether the operation subject predicts the next operation task of the current operation task is determined based on the gaze position change to obtain the task prediction state data, which can help the system understand a task intention of the operation subject to better assist the operation subject in completing the task.

In one embodiment, the performing the gaze position recognition based on the eye movement signal data to obtain the gaze position change of the operation subject includes: determining a gaze point scanning path of the operation subject based on the eye movement signal data; and determining the gaze position change of the operation subject based on the gaze point scanning path.

In this embodiment, the gaze point scanning path of the operation subject is determined based on the eye movement signal data, and the gaze position change of the operation subject is determined based on the gaze point scanning path. In this way, an intention and expected behavior of the operation subject can be estimated.

In this embodiment, whether at least part of the gaze resources of the operation subject is transferred from the current task region range to the next task region range based on the gaze position change is determined to obtain the task prediction state data, which can help the system understand the task intention of the operation subject, so as to determine a state of the operation subject to better guide the operation subject to complete the task.

In one embodiment, the ergonomic data includes at least one of electroencephalogram signal data, electrodermal activity signal data, and heart rate signal data; and the method further includes: performing a load state recognition based on the electroencephalogram signal data to obtain load state data of the operation subject; performing an emotional state recognition based on the electrodermal activity signal data and the heart rate signal data to obtain emotional state data of the operation subject; and performing a prewarning for an operating state of the operation subject based on the load state data and/or the emotional state data.

In this embodiment, the load state recognition is performed based on the electroencephalogram signal data to obtain the load state data of the operation subject, and the emotional state recognition is performed based on the electrodermal activity signal data and the heart rate signal data to obtain the emotional state data of the operation subject. The prewarning is performed on the operating state of the operation subject based on the load state data and/or the emotional state data. Therefore, fatigue, stress, and negative emotions of the operation subject can be discovered and dealt with in time, and operating efficiency, safety, and satisfaction can be improved, thereby avoiding accidents and mistakes during the operation.

In one embodiment, the performing the human-machine collaboration state recognition based on the operation scene image to obtain the target collaboration state data corresponding to the current operation task includes: performing the human-machine collaboration state recognition based on the operation scene image to obtain current collaboration state data of the current operation task as the target collaboration state data, or performing the human-machine collaboration state recognition based on the operation scene image to obtain current collaboration state data of the current operation task, and determining target collaboration state data of an adjacent operation task of the current operation task based on the current collaboration state data.

In this embodiment, the human-machine collaboration state recognition is performed based on the operation scene image to obtain the current collaboration state data of the current operation task as the target collaboration state data, or the human-machine collaboration state recognition is performed based on the operation scene image to obtain the current collaboration state data of the current operation task. The target collaboration state data of the adjacent operation task of the current operation task is determined based on the current collaboration state data, thereby providing important information for the human-machine collaboration process.

In one embodiment, the performing the human-machine collaboration state recognition based on the operation scene image to obtain the target collaboration state data corresponding to the current operation task includes: inputting the operation scene image into a target state recognition model to perform the human-machine collaboration state recognition, to obtain the target collaboration state data.

In this embodiment, the operation scene image is inputted into the target state recognition model to perform the human-machine collaboration state recognition to obtain the target collaboration state data, thereby providing important information for the human-machine collaboration process.

In one embodiment, the target state recognition model is obtained by being trained in following operations: acquiring a sample scene image obtained by shooting a historical operation process, a label of the sample scene image including an annotation task identifier, annotation action data, and an annotation tool category corresponding to the annotation action data; inputting the sample scene image into an initial state recognition model for prediction to obtain a prediction task identifier, prediction action data, and a prediction tool category corresponding to the prediction action data; and updating the initial state recognition model based on the annotation task identifier, the annotation action data, the annotation tool category, the prediction task identifier, the prediction action data, and the prediction tool category to obtain the target state recognition model.

In this embodiment, the sample scene image obtained by shooting the historical operation process is acquired. The sample scene image is inputted into the initial state recognition model for prediction to obtain the prediction task identifier, the prediction action data, and the prediction tool category corresponding to the prediction action data. Based on the annotation task identifier, the annotation action data, the annotation tool category, the prediction task identifier, the prediction action data, and the prediction tool category, the initial state recognition model is updated to obtain the target state recognition model. In this way, the human-machine collaboration state recognition is facilitated based on the operation scene image, and the target collaboration state data corresponding to the current operation task can be known in time.

In one embodiment, the label of the sample scene image is determined by: performing task decomposing on the historical operation process to obtain a plurality of sample operation tasks sorted based on time; determining the annotation task identifier based on task identifiers of the plurality of sample operation tasks; performing encoding on actions involved in the plurality of sample operation tasks to obtain the annotation action data; and performing encoding on tools used when performing the actions involved in the plurality of sample operation tasks to obtain the annotation tool category.

In this embodiment, the task decomposing is performed on the historical operation process to obtain the plurality of sample operation tasks sorted based on time. The annotation task identifier is determined based on the task identifiers of the operation tasks. The annotation action data is obtained by performing the encoding on the actions involved in the sample operation tasks. The annotation tool category is obtained by performing encoding on tools used when executing the actions involved in the sample operation tasks. Thus, actions, tools, and other task information can be effectively represented in a computer device, thereby providing data support for a subsequent training process for the target state recognition model.

In one embodiment, the feedbacking the state of the human-machine collaboration process based on the target collaboration state data and the personnel state data includes: determining preparation state data of a next operation task of the current operation task based on the target collaboration state data, the preparation state data being used to represent a situation that the operation subject needs to prepare in advance for the next operation task; and feedbacking the state of the human-machine collaboration process based on the preparation state data and the personnel state data.

In this embodiment, the preparation state data of the next operation task of the current operation task is determined based on the target collaboration state data, and the state of the human-machine collaboration process is feedbacked based on the preparation state data and the personnel state data. In this way, the human-machine collaboration can be more efficient and reliable.

In one embodiment, the feedbacking the state of the human-machine collaboration process based on the preparation state data and the personnel state data includes: determining a target reminding mode corresponding to the personnel state data; and displaying the preparation state data in the target reminding mode to remind the operation subject to prepare in advance for the next operation task.

In this embodiment, the target reminding mode corresponding to the personnel state data is determined, and the preparation state data is displayed in the target reminding mode to remind the operation subject to prepare in advance for the next operation task, so as to help the operation subject avoid missing key steps or required resources, and to reduce errors and mistakes caused by lack of preparation.

In one embodiment, the method further includes: performing a decision control based on state data feedback for the human-machine collaboration process, to execute a next operation task of the current operation task in an execution mode matching the next operation task. The execution mode is any one of a human-machine collaboration mode, a human-dominated mode, and a machine-dominated mode.

In this embodiment, the decision control is performed based on the state data feedback for the human-machine collaboration process, to execute the next operation task of the current operation task in the execution mode matching the next operation task, which can effectively balance a human decision and a machine decision, so as to improve the efficiency and safety of the collaboration process.

The present disclosure provides an apparatus for feedbacking a human-machine collaboration state based on virtual-real integration. The apparatus includes: a task data acquisition module configured to acquire ergonomic data of an operation subject in a human-machine collaboration process for a current operation task and an operation scene image obtained by shooting the human-machine collaboration process, the operation scene image at least including an operation device and a setting parameter of an operation environment; a human-machine collaboration state recognition module configured to perform a human-machine collaboration state recognition based on the operation scene image to obtain target collaboration state data corresponding to the current operation task; a personnel state recognition module configured to perform a personnel state recognition based on the ergonomic data to obtain personnel state data of the operation subject; and a state feedback module configured to feedback a state of the human-machine collaboration process based on the target collaboration state data and the personnel state data; wherein the ergonomic data comprises eye movement signal data, and the personnel state data comprises task prediction state data; and the personnel state recognition module is further configured to: perform a gaze position recognition based on the eye movement signal data to obtain a gaze position change of the operation subject, wherein the gaze position change is used to describe a change of a gaze position of the operation subject with an execution time of the current operation task; and determine whether the operation subject predicts a next operation task of the current operation task based on the gaze position change to obtain the task prediction state data; wherein the current operation task corresponds to a current task region range, and the next operation task corresponds to a next task region range; and the personnel state recognition module is further configured to: determine whether at least part of gaze resources of the operation subject are transferred from the current task region range to the next task region range based on the gaze position change to obtain the task prediction state data.

The present disclosure provides an electronic device. The electronic device includes: a memory; and one or more processors communicatively coupled to the memory. The memory has instructions executable by the one or more processors stored thereon. The instructions are executable by the one or more processors to enable the one or more processors to perform the method according to any one of the above embodiments.

The present disclosure provides a computer-readable storage medium having a computer program stored thereon. A processor, when executing the computer program, performs the method according to any one of the above embodiments.

The present disclosure provides a computer program product including instructions. The instructions, when executed by a processor of a computer device, enable the computer device to perform the method according to any one of the above embodiments.

In the present disclosure, firstly, the ergonomic data of the operation subject in the human-machine collaboration process for the current operation task, and the operation scene image that is obtained by shooting the human-machine collaboration process and at least includes the operation device and the setting parameter of the operation environment are acquired. Then, the human-machine collaboration state recognition is performed based on the operation scene image to obtain the target collaboration state data corresponding to the current operation task, and the personnel state recognition is performed based on the ergonomic data to obtain the personnel state data of the operation subject, so as to provide important information for the human-machine collaboration process. Finally, based on the target collaboration state data and the personnel state data, the state of the human-machine collaboration process is feedbacked to improve the efficiency and safety of the human-machine collaboration process. The human-machine collaboration system makes corresponding adjustments based on the feedbacked different states to realize a more flexible and adaptive collaboration process. In addition, by feedbacking the state of the human-machine collaboration process, decisions can also be made for subsequent task execution. By optimizing the procedures in the human-machine collaboration process and improving the operating conditions and states of the operation subject, sustainable human-machine collaboration can be achieved.

### BRIEF DESCRIPTION OF THE DRAWINGS

FIG. 1a is a schematic scene diagram of a method for feedbacking a human-machine collaboration state based on virtual-real integration according to an embodiment of the present disclosure.
FIG. 1b is a schematic flowchart of a method for feedbacking a human-machine collaboration state based on virtual-real integration according to an embodiment of the present disclosure.
FIG. 2 is a schematic flowchart of obtaining task prediction state data according to an embodiment of the present disclosure.
FIG. 3a is a schematic flowchart of determining a gaze position change of an operation subject according to an embodiment of the present disclosure.
FIG. 3b is a schematic diagram of a gaze point scanning path according to an embodiment of the present disclosure.
FIG. 4 is a schematic diagram showing a change of eye movement gaze resources along with time according to an embodiment of the present disclosure.
FIG. 5 is a schematic flowchart of performing a prewarning on an operating state of an operation subject according to an embodiment of the present disclosure.
FIG. 6 is a schematic flowchart of obtaining a target state recognition model according to an embodiment of the present disclosure.
FIG. 7a is a schematic flowchart of determining a label of a sample scene image according to an embodiment of the present disclosure.
FIG. 7b is a schematic diagram of performing task decomposing according to an embodiment of the present disclosure.
FIG. 8 is a schematic flowchart of feedbacking a state of a human-machine collaboration process according to an embodiment of the present disclosure.
FIG. 9 is a schematic flowchart of reminding an operation subject to prepare in advance for a next operation task according to an embodiment of the present disclosure.
FIG. 10a is a schematic diagram of performing prewarning feedback on a current task according to an embodiment of the present disclosure.
FIG. 10b is a schematic flowchart of a method for feedbacking a human-machine collaboration state based on virtual-real integration according to an embodiment of the present disclosure.
FIG. 11 is a schematic diagram of an apparatus for feedbacking a human-machine collaboration state based on virtual-real integration according to an embodiment of the present disclosure.
FIG. 12 is an internal structure diagram of an electronic device according to an embodiment of the present disclosure.

### DETAILED DESCRIPTION

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative only and intended to explain, rather than limiting, the present disclosure.

With the development of industrialization, automation, and intelligence, artificial intelligence, virtual reality technology, and augmented reality technology are widely applied in a human-machine collaboration system to promote interaction and collaboration between humans and machines. The embodiments of the present disclosure provide a method for feedbacking a human-machine collaboration state based on virtual-real integration. According to the method for feedbacking the human-machine collaboration state based on virtual-real integration, by using the augmented reality technology, a personnel state recognition and a human-machine collaboration state recognition in a human-machine collaboration process can be performed in real-time, and feedback is made.

In an exemplary embodiment of the present disclosure, firstly, ergonomic data of an operation subject in the human-machine collaboration process for a current operation task, and an operation scene image that is obtained by shooting the human-machine collaboration process and at least includes an operation device and a setting parameter of an operation environment are acquired. Then, a human-machine collaboration state recognition is performed based on the operation scene image to obtain target collaboration state data corresponding to the current operation task, and a personnel state recognition is performed based on the ergonomic data to obtain personnel state data of the operation subject, so as to provide important information for the human-machine collaboration process. Finally, based on the target collaboration state data and the personnel state data, a state of the human-machine collaboration process is feedbacked to improve efficiency and safety of the human-machine collaboration process. A human-machine collaboration system makes corresponding adjustments based on the feedbacked different states to realize a more flexible and adaptive collaboration process. In addition, by feedbacking the state of the human-machine collaboration process, decisions can also be made for subsequent task execution. By optimizing procedures in the human-machine collaboration process and improving operating conditions and states of the operation subject, sustainable human-machine collaboration can be achieved.

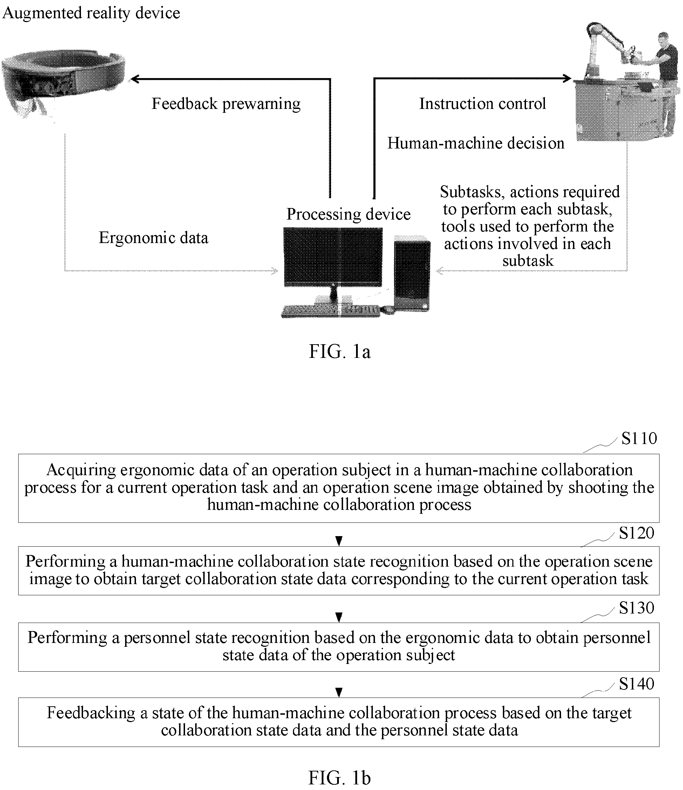

The method for feedbacking the human-machine collaboration state based on virtual-real integration according to the embodiments of the present disclosure may be applied to an augmented reality device. Referring to FIG. 1a, the method for feedbacking the human-machine collaboration state based on virtual-real integration may include an augmented reality module, a state recognition module, and a real-time feedback module. The augmented reality module may be implemented with the augmented reality device. The state recognition module and the real-time feedback module may be deployed on a processing device, such as a computer. Before executing a task A, the processing device may perform a model training by using a plurality of subtasks decomposed from the task A, actions required for executing each subtask, and tools used when executing actions involved in each subtask, to obtain a target state recognition model. On the one hand, the augmented reality device provides scene presentation in a process for executing the task A. On the other hand, a human-machine collaboration scene in a process for executing the task A is collected in real-time by a camera device integrated on the augmented reality device, to obtain the operation scene image at least including the operation device and the setting parameter of the operation environment. In this case, a sensor integrated on the augmented reality device collects eye movement data, physiological data, electroencephalogram signal data, and other data of the operation subject to obtain the ergonomic data of the operation subject. The augmented reality device uploads the collected ergonomic data and the operation scene image to the processing device.

The processing device performs the human-machine collaboration state recognition on the collected operation scene image by using the target state recognition model to obtain target collaboration state data corresponding to a current subtask, and then determine preparation state data of a next subtask of the current subtask based on the target collaboration state data. The processing device performs the personnel state recognition on the collected ergonomic data to obtain the personnel state data of the operation subject. Based on the preparation state data and the personnel state data, the processing device displays the prewarning and a real-time scene of the human-machine collaboration process on an AR interface of the augmented reality device, so as to realize feedback prewarning. The processing device feedbacks the state of the human-machine collaboration process based on the preparation state data and the personnel state data.

To realize human-machine decision-making, an execution mode matching the next subtask of the current subtask is determined based on state data feedback for the human-machine collaboration process, including a human-machine collaboration mode, a human-dominated mode, and a machine-dominated mode. Then, the next subtask is executed according to the determined execution mode to realize instruction control.

The embodiments of the present disclosure provide the method for feedbacking the human-machine collaboration state based on virtual-real integration. Referring to FIG. 1b, the method for feedbacking the human-machine collaboration state based on virtual-real integration may include operations at blocks.

At block S110, ergonomic data of an operation subject in a human-machine collaboration process for a current operation task and an operation scene image obtained by shooting the human-machine collaboration process are acquired.

The operation task may be a complete workflow or a specific subtask or an operation step. For example, the operation task may be one operation step on a factory production line or one link in an airport cargo handling process. The human-machine collaboration process may be a process in which the operation subject and the operation device cooperate to complete a specific task. In the human-machine collaboration process, the operation subject and the operation device cooperate with each other to achieve a common goal through interaction, information exchange, and mutual support. The operation subject may be a human worker performing the current operation task. The ergonomic data may be physiological data and/or behavior data related to the operation subject, such as biological signals such as brain waves, heart rate, and respiration, and behavior information such as action and reaction time. The operation scene image may be an image of the human-machine collaboration process acquired by a shooting device (such as a camera) in the human-machine collaboration process. The operation scene image includes at least the operation device and the setting parameter of the operation environment. The operation device may be a device or machine used by personnel in the human-machine collaboration process to accomplish a specific operation task. The setting parameter of the operation environment may be a parameter set and adjusted in the human-machine collaboration process to ensure correct performance of work.

In an exemplary embodiment of the present disclosure, since the operation device needs to complete some specific tasks together with the operation subject in the human-machine collaboration process, in the human-machine collaboration process of the current task, data collection and data recording are performed through the sensor (such as an electroencephalogram (EEG), a heart rate monitor) or other technical device to obtain the ergonomic data of the operation subject In this way, it helps the operation device better understand and adapt to a state and demand of the operation subject. In addition, in the human-machine collaboration process, the human-machine collaboration process is shot through an image collection device to obtain the operation scene image at least including the operation device and the setting parameter of the operation environment. Therefore, the operation device can perceive and understand a task background, thereby better assisting the operation subject in completing the task. The image collection device can be any one of a camera, a video camera, a fisheye camera, and a monitoring camera.

In some embodiments, a sensor for collecting eye movement data, physiological data, electroencephalogram signal data, and other personal factor data is added to the augmented reality device (such as AR glasses). Therefore, the eye movement data, physiological data, electroencephalogram signal data, and other personal factor data are collected, and the personal factor data of the operation subject in the human-machine collaboration process of the current task is obtained. In this case, real-time collection of human-machine operation scenes is realized through the camera device integrated on the augmented reality device, and the operation scene image at least including the operation device and the setting parameter of the operation environment is obtained.

In some cases, data preprocessing (e.g., noise removal, standardized data format, smoothing) may be performed on the data of the operation subject obtained by the sensor (e.g., the electroencephalogram (EEG), the heart rate monitor) or other technical device and the image captured by the image collection device to obtain the ergonomic data and the operation scene image, so as to improve accuracy and reliability of subsequent analysis.

At block S120, a human-machine collaboration state recognition is performed based on the operation scene image to obtain target collaboration state data corresponding to the current operation task.

The human-machine collaboration state may be a mutual relationship and a cooperation state between the operation subject and the operation device when a collaboration task is performed between the operation subject and the operation device. For example, the human-machine collaboration state may describe, in the specific task, an interaction mode between the operation subject and the operation device and state data of the operation device, etc. The target collaboration state data may be used to describe a cooperation state and behavior between entities (which may be an operation subject, a robot, or other intelligent devices) participating in collaboration. For example, the target collaboration state data may include at least one of tool, action, and interaction information.

In an exemplary embodiment of the present disclosure, feature extraction is performed on the operation scene image to obtain an image feature capable of characterizing the collaboration state. Therefore, the extracted image feature can be predicted to obtain the target collaboration state data, and the human-machine collaboration state recognition can be realized.

For example, the human-machine collaboration state recognition is performed on the operation scene image by using a target detection algorithm to obtain the target collaboration state data corresponding to the current operation task. For example, a pose estimation algorithm may be utilized to classify and identify actions in the operation scene image. A tool recognition algorithm may be used to analyze tools used in the operation scene image to perform the actions involved in the current operation task.

At block S130, a personnel state recognition is performed based on the ergonomic data to obtain personnel state data of the operation subject.

The personal state data may be obtained by observing and analyzing physiological data and behavior data of personnel, so as to obtain information about a current state of the personnel.

In an exemplary embodiment of the present disclosure, feature extraction is performed on the ergonomic data to obtain a feature capable of representing the personnel state. Therefore, the extracted feature can be predicted to obtain the personnel state data, and the personnel state recognition is realized. For example, for different ergonomic data, the personnel state recognition is performed on the ergonomic data by using a corresponding detection method to obtain the personnel state data of the operation subject. For example, the recognition is performed on the basis of the eye movement data of the operation subject, and personnel state data representing a visual attention distribution and a cognitive state of the operation subject is obtained.

At block S140, a state of the human-machine collaboration process is feedbacked based on the target collaboration state data and the personnel state data.

The state feedback may be to feedback on a comprehensive analysis result of the target collaboration state data and the personnel state data, so as to provide corresponding information in time and make corresponding adjustments and decisions.

In an exemplary embodiment of the present disclosure, according to characteristics of different tasks and requirements of human-machine interaction, corresponding state feedback means are designed, such as interface display, voice prompt, vibration prompt, etc. Data analysis is performed on the target collaboration state data and the personnel state data to obtain a data analysis result. Based on key indicators and constraints in the data analysis result, a state of the human-machine collaboration process is determined, such as an actual situation of current task execution. Based on the state of the human-machine collaboration process, a corresponding feedback mean is matched. Feedback information can be timely and accurately transmitted to the operation subject and the operation device in the human-machine collaboration system through the feedback mean.

In some embodiments, AR technology may be used to superimpose the analysis result of the target collaboration state data and the personnel state data in an actual field of view of the operation subject, which can provide more intuitive and real-time information display and decision-making assistance. The operation subject can observe relevant information superimposed in a real scene in real-time through a head-mounted display device (such as AR glasses), so as to better understand the human-machine collaboration state and take corresponding actions.

In other embodiments, the operation subject enters a virtual environment through a VR device (such as a VR headset) and can observe and interact with an object in the virtual environment. The analysis results of the target collaboration state data and the personnel state data are presented in the virtual environment in graphics, animation, or other forms. The operation subject can understand the current state by observing information such as virtual interface or object state, and take corresponding actions.

In the above embodiments, firstly, the ergonomic data of the operation subject in the human-machine collaboration process for the current operation task, and the operation scene image that is obtained by shooting the human-machine collaboration process and at least includes the operation device and the setting parameter of the operation environment are acquired. Then, the human-machine collaboration state recognition is performed based on the operation scene image to obtain the target collaboration state data corresponding to the current operation task, and the personnel state recognition is performed based on the ergonomic data to obtain the personnel state data of the operation subject, so as to provide important information for the human-machine collaboration process. Finally, based on the target collaboration state data and the personnel state data, the state of the human-machine collaboration process is feedbacked to improve the efficiency and safety of the human-machine collaboration process. The human-machine collaboration system makes corresponding adjustments based on the feedbacked different states to realize a more flexible and adaptive collaboration process. In addition, by feedbacking the state of the human-machine collaboration process, decisions can also be made for subsequent task execution. By optimizing the procedures in the human-machine collaboration process and improving the operating conditions and states of the operation subject, sustainable human-machine collaboration can be achieved.

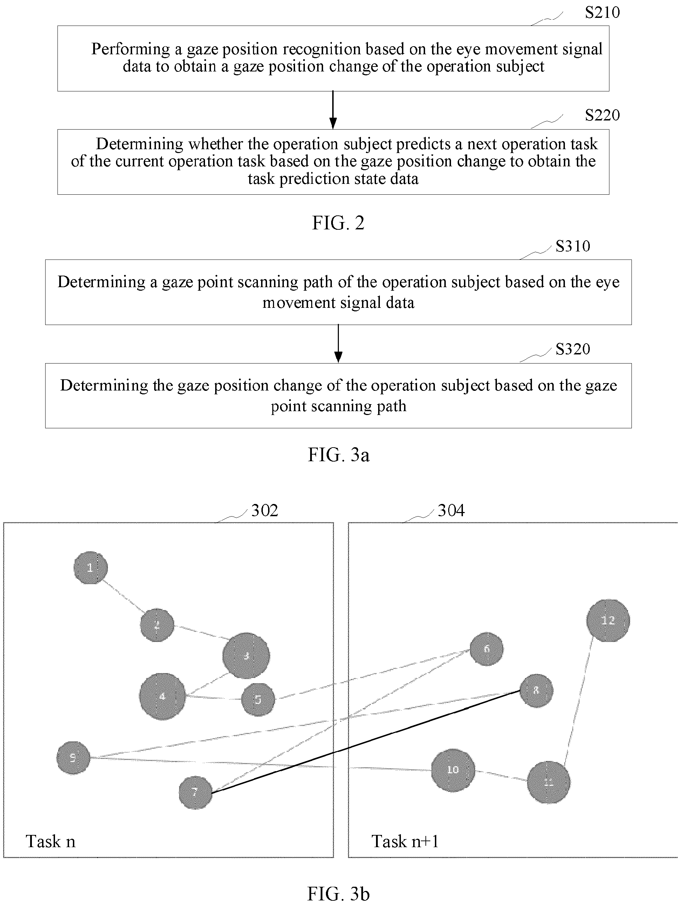

In some embodiments, referring to FIG. 2, the ergonomic data includes eye movement signal data, and the personnel state data includes task prediction state data. The performing the personnel state recognition based on the ergonomic data to obtain the personnel state data of the operation subject may include operations at blocks.

At block S210, a gaze position recognition is performed based on the eye movement signal data to obtain a gaze position change of the operation subject.

At block S220, whether the operation subject predicts a next operation task of the current operation task is determined based on the gaze position change to obtain the task prediction state data.

The eye movement signal data may be data reflecting personnel's eye movement and gaze position acquired through an eye movement tracking technology. The gaze position may be a specific region or a target position to which eyes of the operation subject are directed. The gaze position change is used to describe a change of a gaze position of the operation subject with an execution time of the current operation task. The next operation task may be a next operation task that needs to be completed by the operation subject in a certain order and executed immediately after the current operation task. The task prediction state data may be a representation obtained based on the gaze position change, and is used to describe whether the operation subject predicts the next operation task when executing the current operation task.

In an exemplary embodiment of the present disclosure, the eye movement signal data of the operation subject is recorded by using an eye movement tracking device such as an eye tracker. The eye movement signal data records information such as gaze point coordinates and a gaze duration of the operation subject in the form of time series. Then, the eye movement signal data is processed and analyzed by using analysis algorithms such as a neural network or machine learning to detect the gaze point coordinates, so as to obtain the gaze position of the operation subject. According to the recorded gaze position and in combination with collaboration state information of the current operation task and the next operation task, the gaze position change with time is analyzed, so as to understand the distribution of attention points and attention of the operation subject, and determine whether the operation subject predicts the next operation task. According to a determination result, the task prediction state data is generated. For example, the task prediction state data may be represented as binary indicator variables, such as "predicted" and "non-predicted". When the operation subject has predicted the next operation task of the current operation task, a task prediction state is "predicted", otherwise it is "non-predicted".

In some embodiments, a system for determining the task prediction state data may include an input layer, a recognition layer, and a determination layer. In the input layer, the system inputs in advance the order of each task, a completion duration, a coordinate range of the operation task region, and the gaze position inputted in real-time. The recognition layer is responsible for determining whether the gaze position change with time falls within a region of the current operation task or the next operation task. The determination layer determines, based on the completion time of the operation task and information from the recognition layer, whether the operation subject performs task prediction behavior between completing the current operation task and completing the next operation task, so as to obtain the task prediction state data.

In other embodiments, the predictability of the operation task can be evaluated based on the eye movement gaze and the eye movement scanning path. In this process, considering requirements of a human-machine interaction task, whether information of the next operation task has been noticed can be determined, and when to start paying attention to the next operation task during determining the current operation task can be determined to determine a connection between operation tasks. Then, it can be determined whether the next operation task of the current operation task is predicted according to a duration of the current operation task and the connection between the operation tasks, so as to obtain the task prediction state data.

In the above embodiments, the gaze position recognition is performed based on the eye movement signal data to obtain the gaze position change of the operation subject. Whether the operation subject predicts the next operation task of the current operation task is determined based on the gaze position change to obtain the task prediction state data, which can help the system understand a task intention of the operation subject to better assist the operation subject in completing the task.

In some embodiments, referring to FIG. 3a, the performing the gaze position recognition based on the eye movement signal data to obtain the gaze position change of the operation subject includes operations at blocks.

At block S310, a gaze point scanning path of the operation subject is determined based on the eye movement signal data.

At block S320, the gaze position change of the operation subject is determined based on the gaze point scanning path.

The gaze point scanning path may be a trajectory of a series of gaze points that the eye passes through while observing a specific task or scene during a period of time. The gaze scanning path records a sequence and a path of eye movement from one gaze point to another gaze point.

In an exemplary embodiment of the present disclosure, the eye movement signal data is processed and analyzed by using analysis algorithms such as a neural network or machine learning to detect the gaze point coordinates, so as to obtain the gaze position of the operation subject. By analyzing a shift relationship and duration between gaze positions, the gaze point scanning path is constructed to reveal the attention distribution of the operation subject and a change in the attention distribution. On the basis of the gaze point scanning path, the gaze position change is analyzed. For example, a gaze position and occurrence time of the gaze position in different regions are counted, so as to quantify the attention distribution of the operation subject and infer the attention distribution of the operation subject to different targets.

For example, reference is made to FIG. 3b, FIG. 3b is a schematic diagram of a gaze point scanning path. Twelve gaze positions are included in FIG. 3b and the twelve gaze positions are numbered according to time sequence. A line between every two gaze positions is the gaze point scanning path. According to the gaze point scanning path, it can be determined that gaze positions 1 to 5 are located in a region of task n, gaze positions 5 to 10 switch back and forth between the region of task n and the region of task n+1, and gaze positions 10 to 12 are located in a region of task n+1. Based on the above analysis, the gaze position change can be determined.

In the above embodiment, the gaze point scanning path of the operation subject is determined based on the eye movement signal data, and the gaze position change of the operation subject is determined based on the gaze point scanning path. In this way, an intention and expected behavior of the operation subject can be estimated.

In some embodiments, the current operation task corresponds to a current task region range, and the next operation task corresponds to a next task region range. The determining whether the operation subject predicts the next operation task of the current operation task based on the gaze position change to obtain the task prediction state data includes: determining whether at least part of gaze resources of the operation subject are transferred from the current task region range to the next task region range based on the gaze position change to obtain the task prediction state data

The gaze resources may be the attention distribution of the operation subject on a specific region or target. The task region range may be a spatial range or target region of a particular task that the operation subject is performing, which may be a location or object in a physical environment. The current task region range may be a spatial range or region corresponding to the current task when the current task is executed. The next task region range may be a spatial range or region corresponding to the next task when the next task of the current task is executed.

In an exemplary embodiment of the present disclosure, a current task region range corresponding to a current operation task being executed and a next task region range corresponding to a next operation task are determined. Comparing the gaze position with the task region, whether the gaze position is within the current task region range or within the next task region range is determined. Then, by observing the gaze position change, whether a gaze resource is transferred from the current task region range to the next task region range is determined, and a determination result of the gaze position change is obtained. The task prediction state data is obtained based on the determination result of the gaze position change. For example, the task prediction state data may be represented as binary indicator variables, such as "predicted" and "non-predicted". When the determination result of the gaze position change is that at least part of the gaze resources of the operation subject is transferred from the current task region range to the next task region range, the task prediction state is "predicted", otherwise it is "non-predicted".

In some embodiments, referring to FIG. 3b, a region 302 in FIG. 3b is a current task region range corresponding to the current operation task n being executed. A region 304 in FIG. 3b is a next task region range corresponding to the next operation task n+1. When the gaze positions 5 to 10 are switched back and forth between the task n and the task n+1, it can be determined that at least part of the gaze resources of the operation subject are transferred from the current task region range to the next task region range.

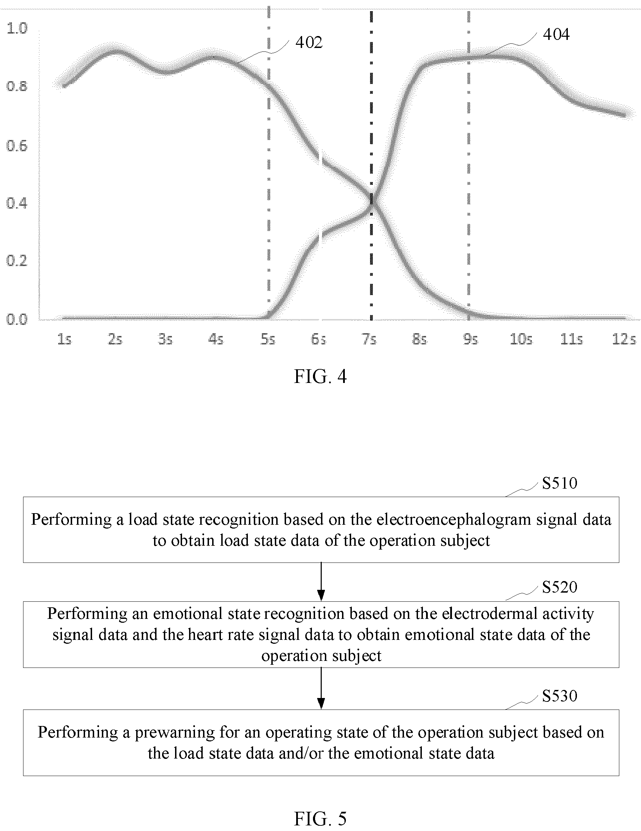

In other embodiments, the current task is denoted as the task n and the next task of the current task is denoted as the task n+1. Referring to FIG. 4, a curve 402 in FIG. 4 shows a change of eye movement gaze resources with time for the task n, and a curve 404 in FIG. 4 shows a change of eye movement gaze resources with time for the task n+1. Assuming that the task n takes 7 seconds to complete, the task n+1 follows the task n. When the task n is currently being performed, the eye gaze resources are concentrated on the task n in a first 5 seconds. Then, starting at the fifth second, some gaze resources are transferred to the next task n+1. Over time, gaze resources tend to be allocated to the task n+1. When the task n ends, there will still be a small part of the gaze resources allocated to the task n because of the continuity of the task.

In the above embodiments, whether at least part of the gaze resources of the operation subject is transferred from the current task region range to the next task region range based on the gaze position change is determined to obtain the task prediction state data, which can help the system understand the task intention of the operation subject, so as to determine a state of the operation subject to better guide the operation subject to complete the task.

In some embodiments, referring to FIG. 5, the ergonomic data includes at least one of electroencephalogram signal data, electrodermal activity signal data, and heart rate signal data. The method may further include operations at blocks.

At block S510, a load state recognition is performed based on the electroencephalogram signal data to obtain load state data of the operation subject.

The electroencephalogram signal data may be an electrical signal for detecting and recording brain activity of the operation subject by placing electrodes on a scalp surface. A load state may be a degree of psychological and physiological stress experienced by the operation subject while performing one task. The load state data may be a record or representation of the load state of the operation subject obtained by a load state recognition method based on the electroencephalogram signal data.

In an exemplary embodiment of the present disclosure, the load state is classified into different categories according to a preset determination criterion. This indicates that there is a well-defined criterion to evaluate and distinguish between different load states. Based on the determination criterion and different categories of load states, corresponding load state models are established. By using an electroencephalogram signal collection device (such as an electroencephalogram (EEG) device), the electroencephalogram signal data of the operation subject is collected. Then, the load state model is used to recognize the load state of electroencephalogram signal data to obtain the load state of the operation personnel when performing the task. Support vector machine and machine learning algorithm are used to realize the load state recognition, which can effectively distinguish different categories of the load states. According to real-time evaluation based on load modeling, a working state of the operation personnel can be accurately monitored and adjusted to improve productivity and work quality.

For example, according to α/β value of the EEG as the determination criterion, the load state is divided into high, medium, and low three cases, and the corresponding load state models are established. By monitoring a prefrontal electroencephalogram signal of the operation personnel, prefrontal electroencephalogram signal data is obtained. Through the load state model, the prefrontal electroencephalogram signal data can be recognized, and the load state of the operation personnel during the task can be obtained.

At block S520, an emotional state recognition is performed based on the electrodermal activity signal data and the heart rate signal data to obtain emotional state data of the operation subject.

The electrodermal activity signal data may be an electrophysiological response of a skin surface. The electrodermal activity signal data is usually associated with an emotional state such as anxiety, excitement, and stress. The heart rate signal data may measure a change in the heartbeat of the operation subject. The heart rate signal data may reflect the rhythmic activity of the heart. The emotional state may be a subjective emotion experienced by the operation subject at a particular moment in time. The emotional state data may be information describing a current emotional state of the operation subject obtained by analyzing the electrodermal activity signal data and the heart rate signal data.

In some cases, the electrodermal activity signal data and the heart rate signal data can reflect sympathetic nerve activity, and the sympathetic nerve activity has some correlation with the emotional state. Therefore, the information reflecting the emotional state of the operation subject can be acquired by monitoring the electrodermal activity signal data and the heart rate signal data of the operation subject.

In an exemplary embodiment of the present disclosure, the electrodermal activity signal data of the operation subject is collected by using an electrodermal sensor, and the heart rate signal data of the operation subject is collected by using a heart rate monitoring device. A machine learning algorithm, such as a support vector machine (SVM), a decision tree, a neural network, etc., and a deep learning method can be used to build the emotional state model. In a process of emotional state recognition, the electrodermal activity signal data and the heart rate signal data are inputted into the emotional state model. According to a state classification of the emotional state models, the electrodermal activity signal data and the heart rate signal data are recognized to obtain the emotional state data of the operation subject. An abnormal emotional state of the operation subject can be recognized based on the emotional state data of the operation subject. An abnormal emotional state recognition may be performed based on the electrodermal activity signal data and the heart rate signal data to obtain the emotional state data of the operation subject. It should be noted that the heart rate signal data may also be an LF index (low frequency component) of the heart rate variability.

In some embodiments, an emotional baseline state is measured and recorded for each operation subject, and a baseline state database corresponding to the operation subjects is established. In this process, a physiological signal such as the electrodermal activity signal data and the heart rate signal data can be used to measure the emotional baseline state of each operation subject. A baseline state X value of each operation personnel is acquired from the baseline state database as a reference value for determining emotional abnormality. An actual state Y value of the operation subject is compared with the baseline state X value, and if Y>aX, the emotional abnormality is determined to exist. A threshold a can be customized according to actual needs, and the threshold a can be determined by experiment or experience.

At block S530, a prewarning is performed for an operating state of the operation subject based on the load state data and/or the emotional state data.

In an exemplary embodiment of the present disclosure, a prewarning index and a threshold corresponding to the load state data and the emotional state data are set according to actual needs, and are used for determining whether the operation state of the operation subject is abnormal. The load state data and/or the emotional state data of the operation subject are monitored in real-time, and determined according to the set prewarning index or the threshold. When the load state data and/or the emotional state data of the operation subject conform to the prewarning index or exceed the threshold, a prewarning mechanism is triggered to prewarn the operation state of the operation subject. Further, prewarning information can be sent to relevant personnel, such as the operation subject, a supervisor, etc. In addition, different prewarning methods can be adopted, such as sound, vibration, flash, etc., in order to attract attention in time.

In the above embodiments, the load state recognition is performed based on the electroencephalogram signal data to obtain the load state data of the operation subject, and the emotional state recognition is performed based on the electrodermal activity signal data and the heart rate signal data to obtain the emotional state data of the operation subject. The prewarning is performed on the operating state of the operation subject based on the load state data and/or the emotional state data. Therefore, fatigue, stress, and negative emotions of the operation subject can be discovered and dealt with in time, and operating efficiency, safety, and satisfaction can be improved, thereby reducing occurrence of accidents and mistakes during the operation.

It should be noted that, based on the task prediction state data, the attention of the operation subject and the performance of the operation subject can be evaluated. For example, when the operation subject is under high load, mentally fatigued, or emotionally abnormal, the task prediction state data of the operation subject can be determined based on preparation state data. By analyzing the task prediction state data, whether the operation subject allocates attention resources to the next operation task can be determined. When the operation subject can efficiently allocate the attention resources to the next operation task, then the operation subject can be considered as being suitable for working in a high-intensity work environment. When the operation subject does not allocate the attention resources to the next operation task, the operation subject can be considered as being unsuitable for the high-intensity work environment.

In addition, when the operation subject waits for a long time, a distracted state may occur. According to the task prediction state data, whether the operation subject allocates the attention resources to the next operation task can be determined. When the operation subject can allocate the attention resources to the next operation task, the operation subject can be considered as being suitable for work in a work environment that requires high concentration.

When an unexpected situation occurs, the required prediction state data can be acquired. By using the prediction state data required in the unexpected situation, the preparation state data of the operation subject can be determined. Whether the operation subject allocates the attention resources to the unexpected situation is determined based on the preparation state data. When the operation subject can effectively allocate the attention resources to deal with the unexpected situation, the operation subject can be considered to have an ability to deal with the unexpected situation. When the operation subject fails to allocate the attention resources to deal with the unexpected situation and the operation subject is not under high load or fatigue, the operation subject can be considered to lack the ability to deal with complex tasks.

In some embodiments, the performing the human-machine collaboration state recognition based on the operation scene image to obtain the target collaboration state data corresponding to the current operation task may include: performing the human-machine collaboration state recognition based on the operation scene image to obtain current collaboration state data of the current operation task as the target collaboration state data.

In an exemplary embodiment of the present disclosure, the feature extraction is performed on the operation scene image to obtain the image feature capable of characterizing the collaboration state. Therefore, the extracted image feature can be predicted to obtain the current collaboration state data of the current operation task, such as information such as a state of tools, a relative position between tools, an interaction action between the operation subject and the operation device, etc. In order to monitor the current collaboration state data in real-time and feedback correspondingly to reduce errors and delays, the current collaboration state data can be taken as target collaboration state data.

In the above embodiments, the human-machine collaboration state recognition is performed based on the operation scene image to obtain the current collaboration state data of the current operation task as the target collaboration state data, thereby providing important information for the human-machine collaboration process.

In some embodiments, the performing the human-machine collaboration state recognition based on the operation scene image to obtain the target collaboration state data corresponding to the current operation task may include: performing the human-machine collaboration state recognition based on the operation scene image to obtain current collaboration state data of the current operation task; and determining target collaboration state data of an adjacent operation task of the current operation task based on the current collaboration state data.

A task decomposing is performed on the operation process to obtain task requirement data. The task requirement data includes a plurality of operation tasks sorted based on time. The task requirement data includes the current operation task, which correspondingly has the adjacent operation task. For example, the adjacent operation task may be an operation task arranged before and adjacent to the current operation task in the task requirement data. The adjacent operation task may be an operation task arranged after and adjacent to the current operation task in the task requirement data. It should be noted that a task execution order in the task requirement data predetermined may be different from a task execution order in the actual operation process.

In an exemplary embodiment of the present disclosure, the feature extraction is performed on the operation scene image to obtain the image feature capable of characterizing the collaboration state. Therefore, the extracted image feature can be predicted to obtain the current collaboration state data of the current operation task. Then, according to a task ordering in the task requirement data, the adjacent operation task of the current operation task is found by using the current collaboration state data. After the adjacent operation task is determined, the collaboration state data corresponding to the adjacent operation task can be searched for from a storage unit and taken as the target collaboration state data. The target detection algorithm may also be used to perform the collaboration state recognition of the real-time operation scene image to obtain the collaboration state data of the adjacent operation task of the current operation task, and take the collaboration state data of the adjacent operation task as the target collaboration state data.

For example, the task requirement data may include an operation task 1, an operation task 2, and an operation task 3 arranged in execution order. However, in an actual execution process, when a currently executed operation task is the operation task 2, since the operation task1 is not executed, the adjacent operation task 1 can be used as the next operation task. The collaboration state data of the operation task 1 may be used as the target collaboration state data. When the currently executed operation task is the operation task 2, since the operation task 1 has already been executed, the adjacent operation task 3 can be used as the next operation task. The collaboration state data of the operation task 3 can be used as the target collaboration state data.

In the above embodiments, the human-machine collaboration state recognition is performed based on the operation scene image to obtain the current collaboration state data of the current operation task. The target collaboration state data of the adjacent operation task of the current operation task is determined based on the current collaboration state data, thereby providing important information for the human-machine collaboration process.

In some embodiments, the performing the human-machine collaboration state recognition based on the operation scene image to obtain the target collaboration state data corresponding to the current operation task may include: inputting the operation scene image into a target state recognition model to perform the human-machine collaboration state recognition, to obtain the target collaboration state data.

In an exemplary embodiment of the present disclosure, an initial state recognition model can be built in advance, and the initial state recognition model is trained to obtain the target state recognition model. The target state recognition model is deployed in the processing device. The processing device is usually connected to the image collection device. The operation scene image is obtained by shooting the operation scene through the image collection device. The image collection device sends the operation scene image to the processing device, and the processing device takes the operation scene image as an input of the target state recognition model. The feature extraction is performed, by the target state recognition model, on the operation scene image to obtain the image feature capable of representing the collaboration state. In this way, the prediction can be performed based on the extracted image feature, and the target state recognition model can output the target collaboration state data to realize the human-machine collaboration state recognition. It can be understood that, in some cases, the target state recognition model may further be deployed in the image collection device, and the operation scene image is obtained by shooting the operation scene by the image collection device, and the operation scene image is inputted to the target state recognition model to perform the human-machine collaboration state recognition to obtain the target collaboration state data.

In the above embodiments, the operation scene image is inputted into the target state recognition model to perform the human-machine collaboration state recognition to obtain the target collaboration state data, thereby providing important information for the human-machine collaboration process.

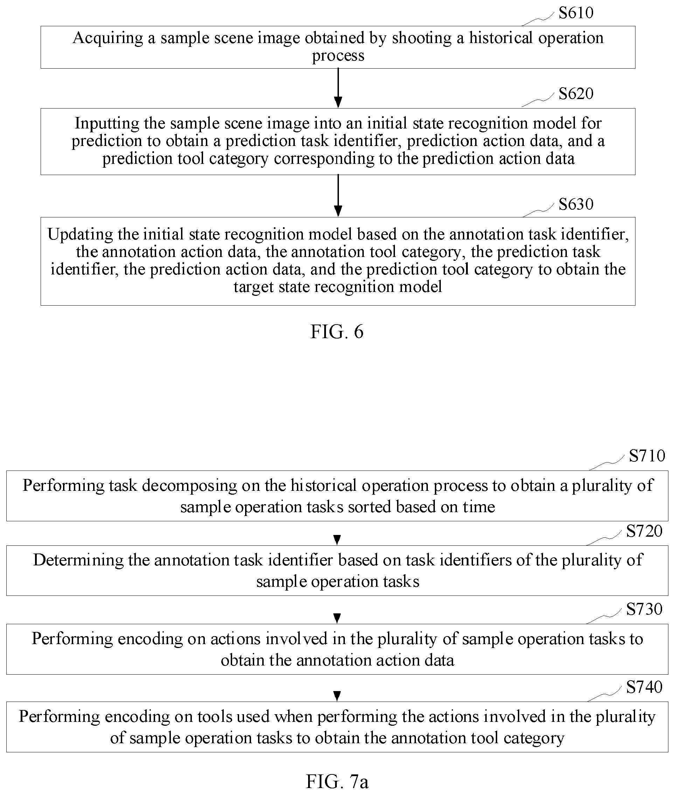

In some embodiments, referring to FIG. 6, the target state recognition model is obtained by being trained in following operations.

At block S610, a sample scene image obtained by shooting a historical operation process is acquired.

At block S620, the sample scene image is inputted into an initial state recognition model for prediction to obtain a prediction task identifier, prediction action data, and a prediction tool category corresponding to the prediction action data.

At block S630, the initial state recognition model is updated based on the annotation task identifier, the annotation action data, the annotation tool category, the prediction task identifier, the prediction action data, and the prediction tool category to obtain the target state recognition model.

A label of the sample scene image may include an annotation task identifier, annotation action data, and an annotation tool category corresponding to the annotation action data. The task identifier can be used to identify a specific task or operation type. The action data can be used to characterize actions required to perform the operation task. The tool category can be used to characterize tools used to execute the operation task.

In an exemplary embodiment of the present disclosure, the sample scene image is obtained by shooting the historical operation process using the image collection device. By using a professional annotation tool, the sample scene image is annotated, and the corresponding annotation task identifier, annotation action data, annotation tool category are assigned to each frame of the sample scene image, so as to obtain the label of the sample scene image. The sample scene image is inputted to the initial state recognition model for prediction, to obtain the prediction task identifier, the prediction action data, and the prediction tool category corresponding to the prediction action data. A first loss value is then determined based on the annotation task identifier and the prediction task identifier. A second loss value is determined based on the annotation action data and the prediction action data. A third loss value is determined based on the annotation tool category and the prediction tool category. The initial state recognition model is updated based on the first loss value, the second loss value, and the third loss value. By analogy, the updated initial state recognition model is continuously trained, and when a model training stop condition is met, the target state recognition model can be obtained. The model training stop condition may be that a model loss value tends to converge, or that training rounds reach a preset number of rounds.

In some embodiments, the initial state recognition model may employ a YOLO V5 model. The YOLO V5 model is a neural network model algorithm used for object detection task, and a main goal of this model is to recognize objects present in an image and determine a position of the recognized object on the image. The model divides the image into an S×S grid, and each grid cell is responsible for detecting objects. By predicting for each grid cell, a position of a bounding box, a confidence score of the box, and category probability of the grid cell containing the object can be obtained. Then, an output result of the model can be determined by performing data analysis based on the position of the bounding box, the confidence score of the box, and the category probability of the grid cell containing the object, and then the object detection task can be realized.

In the above embodiment, the sample scene image obtained by shooting the historical operation process is acquired. The sample scene image is inputted into the initial state recognition model for prediction to obtain the prediction task identifier, the prediction action data, and the prediction tool category corresponding to the prediction action data. Based on the annotation task identifier, the annotation action data, the annotation tool category, the prediction task identifier, the prediction action data, and the prediction tool category, the initial state recognition model is updated to obtain the target state recognition model. In this way, the human-machine collaboration state recognition is facilitated based on the operation scene image, and the target collaboration state data corresponding to the current operation task can be known in time.

In some embodiments, referring to FIG. 7a, the label of the sample scene image is determined by operations at blocks.

At block S710, task decomposing is performed on the historical operation process to obtain a plurality of sample operation tasks sorted based on time.

At block S720, the annotation task identifier is determined based on task identifiers of the plurality of sample operation tasks.

At block S730, encoding is performed on actions involved in the plurality of sample operation tasks to obtain the annotation action data.

At block S740, encoding is performed on tools used when performing the actions involved in the plurality of sample operation tasks to obtain the annotation tool category.

In an exemplary embodiment of the present disclosure, an appropriate task decomposing mode is selected based on factors such as nature, complexity, and key nodes in the execution process of the operation task. According to the determined task decomposing mode, the task decomposing is performed on the historical operation process to obtain the plurality of sample operation tasks sorted based on time. Each sample operation task correspondingly has information such as an action and a tool used to execute the action involved in the sample operation task. Each sample operation task is independent of each other. That is, there is an execution sequence among sample operation tasks, but there is no overlapping part in space, which can also be understood as actions and tools for completing each sample operation task are independent of each other. Each sample operation task is assigned a unique task identifier, such as a number or name. A unique annotation task identifier for each sample operation task is assigned based on the task identifier of the sample operation task. The task identifier of the sample operation task can be directly used as the annotation task identifier, or the annotation task identifier can be obtained by encoding the task identifier of the sample operation task. The actions involved in the sample operation task are encoded, and the actions involved in each sample operation task are represented as unique annotation action data. In this way, each action can be accurately identified and recorded in subsequent processing and analysis. The encoding is performed on the tools used when performing the actions involved in the sample operation task, and each tool used when performing the actions involved in the sample operation task is represented as a unique annotation tool category. In this way, each tool can be accurately identified and recorded in subsequent processing and analysis.

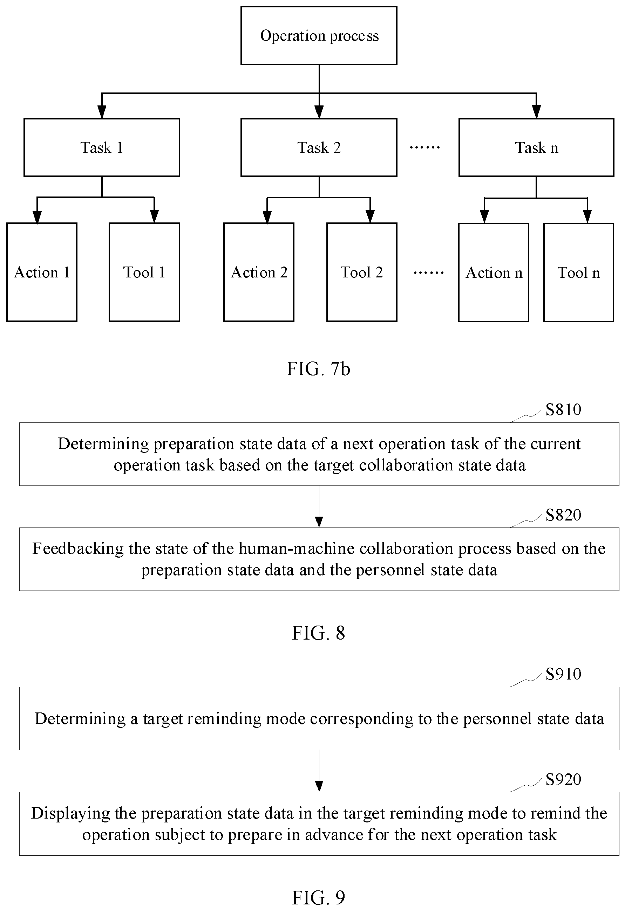

For example, according to the task of the operation device and the task type of the task that the operation subject needing to operate, the task can be distinguished based on requirements of time series, and the operation process needing to be completed is decomposed into a series of tasks. First, for the operation process needing to be completed, components of the task can be analyzed based on the human-machine collaboration process, and the components include the actions involved in the task and the tools needed to complete the actions. Further, the operation process can be decomposed into a series of tasks, and the actions required to perform each task and the tools used to perform the actions involved in each task can be determined. In the task execution process, the tools used to perform the actions involved in each task are used to complete the required actions at defined locations, so as to achieve the execution for the task. Referring to FIG. 7b, the operation process can be decomposed into task 1, task 2...task n, and each task consists of corresponding actions and tools. For example, components of the task 1 includes an action 1 required for performing the task 1 and a tool 1 used for performing the action 1 involved in the task 1; components of the task 2 includes an action 2 required for performing the task 2 and a tool 2 used for performing the action 2 involved in the task 2; and components of the task n includes an action n required for performing the task n and a tool n used for performing the action n involved in the task n.

In the above embodiments, the task decomposing is performed on the historical operation process to obtain the plurality of sample operation tasks sorted based on time. The annotation task identifier is determined based on the task identifiers of the operation tasks. The annotation action data is obtained by performing the encoding on the actions involved in the sample operation tasks. The annotation tool category is obtained by performing encoding on tools used when executing the actions involved in the sample operation tasks. Thus, actions, tools, and other task information can be effectively represented in a computer device, thereby providing data support for a subsequent training process for the target state recognition model.

In some embodiments, referring to FIG. 8, the feedbacking the state of the human-machine collaboration process based on the target collaboration state data and the personnel state data may include operations at blocks.

At block S810, preparation state data of a next operation task of the current operation task is determined based on the target collaboration state data.

At block S820, the state of the human-machine collaboration process is feedbacked based on the preparation state data and the personnel state data.

The preparation state data is used to represent a situation that the operation subject needs to prepare in advance for the next operation task. The preparation state data may be information and requirements related to the next operation task, and the information and requirements need to be prepared in advance before completing the current operation task. For example, the preparation state data may include at least one of material, device, tool, operation step, sequence, precaution, and the like required for the next operation task.

In an exemplary embodiment of the present disclosure, in response to determining the target collaboration state data of the current operation task, the target state recognition model can recognize the next operation task of the current operation task and the collaboration state data of the next operation task. The collaboration state data of the next operation task is analyzed to obtain the preparation state data required by the next operation task. The preparation state data may include at least one of material, step description, and related information required to execute the next operation task. The personal state data may include information on physiological state, attention level, and the like of the operation subject. By analyzing the preparation state data and the personnel state data, various state information in the human-machine collaboration process can be known in time, and can be feedbacked to the operation subject and/or the operation device in an appropriate way, which helps the operation subject to adjust a strategy or decision in time, and also helps the operation device to adjust its own operation state based on the feedback information, so as to better adapt to current collaboration requirements and a current environment.

For example, when the personnel state data indicates that the operation subject is in an abnormal state (such as a fatigue state), and the execution mode of the next operation task can be the human-dominated mode or the machine-dominated mode. Then, when the personnel state data indicates that the operation subject is in the abnormal state, the preparation state data is sent to the operation device, and the next operation task is executed in the machine-dominated mode, which can reduce operation risks of the operation subject in the fatigue state and improve the safety and reliability of the task execution.

In the above embodiments, the preparation state data of the next operation task of the current operation task is determined based on the target collaboration state data, and the state of the human-machine collaboration process is feedbacked based on the preparation state data and the personnel state data. In this way, the human-machine collaboration can be more efficient and reliable.

In some embodiments, referring to FIG. 9, the feedbacking the state of the human-machine collaboration process based on the preparation state data and the personnel state data may include operations at blocks.

At block S910, a target reminding mode corresponding to the personnel state data is determined.

At block S920, the preparation state data is displayed in the target reminding mode to remind the operation subject to prepare in advance for the next operation task.

The target reminding mode may be to convey the target and preparation state data of the next operation task to the operation subject through a specific way and mean, so as to prepare in advance. For example, the target reminding mode may be a visual display or a voice prompt.

In an exemplary embodiment of the present disclosure, the personnel state data includes a concentration degree, a fatigue degree, and the emotional state of the operation subject, the personnel state data is analyzed and organized to obtain an analysis result of the personnel state data. According to the analysis result of the personnel state data, the appropriate target reminding mode (such as the visual display, the voice prompt, vibration) is determined, so as to convey the preparation state data of the next operation task to the operation subject, thus reminding the operation subject to prepare for the next operation task in advance. Based on the selected target reminding mode, the preparation state data is displayed to the operation subject in an appropriate form, so that the operation subject can timely receive and understand the preparation state data.

For example, when the personnel state data indicates that the operation subject is not in the abnormal state, and the execution mode of the next operation task is the human-dominated mode, then, the visual display mode can be selected to display the relevant preparation state data on an operation interface to attract the attention of the operation subject. Therefore, the operation subject can obtain necessary information in time and dominate the execution of the next operation task.

When the personnel state data indicates that the operation subject is in the abnormal state (for example, the task prediction state data of the operation subject is non-predicted or in the fatigued state), and the execution mode of the next operation task is the human-dominated mode, then, the operation subject can be stimulated by means of strong reminders (such as the voice prompt) to attract the attention of the operation subject to the preparation state data. The operation subject can readjust attention and concentration to dominate the execution of the next operation task.

In some embodiments, the augmented reality technology may be used to display preparation state data such as task objectives and preparation requirements to the operation subject by superimposing text, images, videos, etc. in the real scene. The operation subject obtains the preparation state data in the AR interface through the augmented reality device (such as AR glasses), which helps the operation subject make necessary preparations and adjustments to ensure the smooth execution of the task.

In other embodiments, the virtual reality technology is utilized to create elements, such as virtual objects, text, audio, in the virtual environment to present the preparation state data. The operation subject can obtain the preparation state data in an VR interface through a virtual reality device (such as a VR headset).

In the above embodiments, the target reminding mode corresponding to the personnel state data is determined, and the preparation state data is displayed in the target reminding mode to remind the operation subject to prepare in advance for the next operation task, so as to help the operation subject avoid missing key steps or required resources, and to reduce errors and mistakes caused by lack of preparation.

In some embodiments, the method may further include: performing a decision control based on state data feedback for the human-machine collaboration process, to execute a next operation task of the current operation task in an execution mode matching the next operation task.

The execution mode is any one of a human-machine collaboration mode, a human-dominated mode, and a machine-dominated mode. The human-machine collaboration mode may be a mode in which the human and the machine cooperate and work together in the human-machine collaboration process. The human-dominated mode may be a mode in which the human plays a dominant role in a decision-making and control process, and a way of the machine performing the task depends on human instructions. The machine-dominated mode may be a mode in which the machine plays a dominant role in the decision-making and control process, while the human plays a supportive role throughout the human-machine collaboration process.

In an exemplary embodiment of the present disclosure, in the human-machine collaboration process, a situation of the next operation task is evaluated through the state data feedback by the human-machine collaboration process, to determine the execution mode matching the next operation task. When the next operation task requires a high degree of human-machine collaboration, then the human-machine collaboration mode is selected. When the next operation task requires the operation subject to guide and make decisions, the human-dominated mode is selected. When the next operation task can be performed autonomously by the operation device, the machine-dominated mode is selected. According to the selected execution mode, a corresponding execution plan is formulated. In the human-machine collaboration mode, taking into account advantages and limitations of both the human and the machine, the operation subject and the operation device need to work together to formulate an appropriate execution strategy. In the human-dominated mode, the operation subject needs to formulate the execution plan based on state data and professional knowledge, and task instructions for the execution plan are conveyed to the operation device. In the machine-dominated mode, the operation device needs to independently formulate the execution plan according to a built-in algorithm and a rule system, without the intervention of the operation subject. In response to determining the execution plan, the next operation task is executed according to the formulated execution plan.

In some embodiments, according to the selected execution mode, the augmented reality device is utilized to superimpose appropriate virtual information in the real scene in which the operation subject is located, so as to help the operation subject perform the operation task better. For example, in the human-machine collaboration mode, an operation state and a task progress of the operation device can be superimposed and displayed in the real scene. In this way, the operation subject can adjust the strategy in time. In the human-dominated mode, a task execution instruction and an operation flow can be superimposed and displayed in the real scene to help the operation subject better complete the operation task. In the machine-dominated mode, an execution status and an execution result of the operation device can be superimposed and displayed in the real scene. In this way, the operation subject can understand the operating state and data of the operation device in time.

In other embodiments, the virtual reality device is utilized to create a corresponding virtual environment according to the selected execution mode. In the virtual environment, an interaction process between the operation subject and the operation device can be simulated, and information such as the task execution instruction, the operation flow, and a machine state can be displayed. For example, in the human-dominated mode, the operation subject can execute the next operation task based on information and guidance displayed in the virtual environment.

In the above embodiments, the decision control is performed based on the state data feedback for the human-machine collaboration process, to execute the next operation task of the current operation task in the execution mode matching the next operation task, which can effectively balance a human decision and a machine decision, so as to improve the efficiency and safety of the collaboration process.

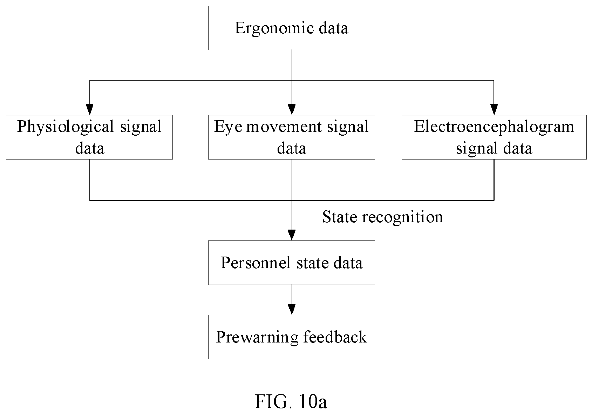

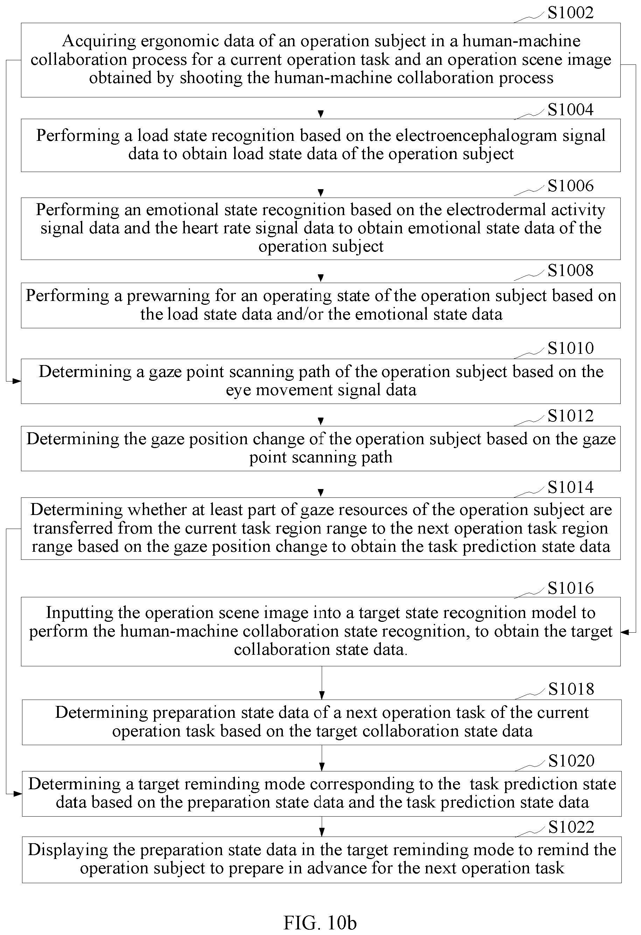

The embodiments of the present disclosure provide the method for feedbacking the human-machine collaboration state based on virtual-real integration. Referring to FIG. 10a, the ergonomic data includes physiological signal data (such as the electrodermal activity signal data and the heart rate signal data), the eye movement signal data, and electroencephalogram signal data. The personnel state recognition may be performed based on the physiological signal data, the eye movement signal data, and the electroencephalogram signal data to obtain the personnel state data. For example, the personnel state data may include the task prediction state data. It can be understood that prewarning feedback can be performed on the current operation task based on the personnel state data. The current operation task corresponds to the current task region range, and the next operation task corresponds to the next task region range. For example, referring to FIG. 10b, the method for feedbacking the human-machine collaboration state based on virtual-real integration may include operations at blocks.

At block S1002, ergonomic data of an operation subject in a human-machine collaboration process for a current operation task and an operation scene image obtained by shooting the human-machine collaboration process are acquired.

The operation scene image at least includes the operation device and the setting parameter of the operation environment.

At block S1004, a load state recognition is performed based on the electroencephalogram signal data to obtain load state data of the operation subject.

At block S1006, an emotional state recognition is performed based on the electrodermal activity signal data and the heart rate signal data to obtain emotional state data of the operation subject.

At block S1008, a prewarning is performed for an operating state of the operation subject based on the load state data and/or the emotional state data.

At block S1010, a gaze point scanning path of the operation subject is determined based on the eye movement signal data.

At block S1012, the gaze position change of the operation subject is determined based on the gaze point scanning path.

The gaze position change is used to describe the change of the gaze position of the operation subject with the execution time of the current operation task.

At block S 1014, whether at least part of gaze resources of the operation subject are transferred from the current task region range to the next task region range is determined based on the gaze position change to obtain the task prediction state data.

At block S1016, the operation scene image is inputted into a target state recognition model to perform the human-machine collaboration state recognition, to obtain the target collaboration state data.

At block S1018, preparation state data of a next operation task of the current operation task is determined based on the target collaboration state data.

The preparation state data is used to represent the situation that the operation subject needs to prepare in advance for the next operation task.

At block S1020, a target reminding mode corresponding to the task prediction state data is determined based on the preparation state data and the task prediction state data.

At block S1022, the preparation state data is displayed in the target reminding mode to remind the operation subject to prepare in advance for the next operation task.

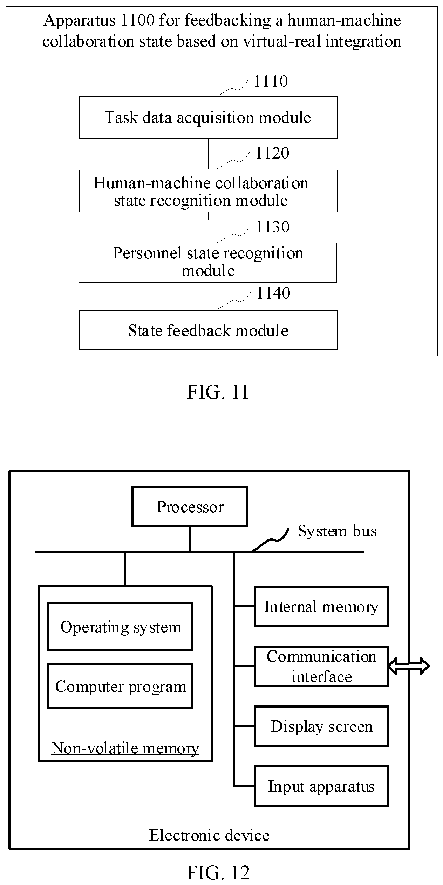

The embodiments of the present disclosure provide an apparatus 1100 for feedbacking a human-machine collaboration state based on virtual-real integration. Referring to FIG. 11, the apparatus 1100 includes a task data acquisition module 1110, a human-machine collaboration state recognition module 1120, a personnel state recognition module 1130, and a state feedback module 1140.

The task data acquisition module 1110 is configured to acquire ergonomic data of an operation subject in a human-machine collaboration process for a current operation task and an operation scene image obtained by shooting the human-machine collaboration process. The operation scene image at least includes an operation device and a setting parameter of an operation environment.

The human-machine collaboration state recognition module 1120 is configured to perform a human-machine collaboration state recognition based on the operation scene image to obtain target collaboration state data corresponding to the current operation task.

The personnel state recognition module 1130 is configured to perform a personnel state recognition based on the ergonomic data to obtain personnel state data of the operation subject.

The state feedback module 1140 is configured to feedback a state of the human-machine collaboration process based on the target collaboration state data and the personnel state data.

In some embodiments, the ergonomic data includes eye movement signal data, and the personnel state data includes task prediction state data. The personnel state recognition module is further configured to: perform a gaze position recognition based on the eye movement signal data to obtain a gaze position change of the operation subject, the gaze position change being used to describe a change of a gaze position of the operation subject with an execution time of the current operation task; and determine whether the operation subject predicts a next operation task of the current operation task based on the gaze position change to obtain the task prediction state data.

In some embodiments, the apparatus for feedbacking the human-machine collaboration state based on virtual-real integration further includes a gaze position change module configured to determine a gaze point scanning path of the operation subject based on the eye movement signal data and determine the gaze position change of the operation subject based on the gaze point scanning path.

In some embodiments, the current operation task corresponds to a current task region range, and the next operation task corresponds to a next task region range. The personnel state recognition module is further configured to determine whether at least part of gaze resources of the operation subject are transferred from the current task region range to the next task region range based on the gaze position change to obtain the task prediction state data.

In some embodiments, the ergonomic data includes at least one of the electroencephalogram signal data, electrodermal activity signal data, and heart rate signal data. The apparatus for feedbacking the human-machine collaboration state based on virtual-real integration further includes a working state prewarning module configured to: perform a load state recognition based on the electroencephalogram signal data to obtain load state data of the operation subject; perform an emotional state recognition based on the electrodermal activity signal data and the heart rate signal data to obtain emotional state data of the operation subject; and perform a prewarning for an operating state of the operation subject based on the load state data and/or the emotional state data.

In some embodiments, the human-machine collaboration state recognition module is further configured to: perform the human-machine collaboration state recognition based on the operation scene image to obtain current collaboration state data of the current operation task as the target collaboration state data, or perform the human-machine collaboration state recognition based on the operation scene image to obtain current collaboration state data of the current operation task, and determining target collaboration state data of an adjacent operation task of the current operation task based on the current collaboration state data.

In some embodiments, the human-machine collaboration state recognition module is further configured to input the operation scene image into a target state recognition model to perform the human-machine collaboration state recognition, to obtain the target collaboration state data.

In some embodiments, the target state recognition model is obtained by being trained in following operations: acquiring a sample scene image obtained by shooting a historical operation process, a label of the sample scene image including an annotation task identifier, annotation action data, and an annotation tool category corresponding to the annotation action data; inputting the sample scene image into an initial state recognition model for prediction to obtain a prediction task identifier, prediction action data, and a prediction tool category corresponding to the prediction action data; and updating the initial state recognition model based on the annotation task identifier, the annotation action data, the annotation tool category, the prediction task identifier, the prediction action data, and the prediction tool category to obtain the target state recognition model.

In some embodiments, the label of the sample scene image is determined by: performing task decomposing on the historical operation process to obtain a plurality of sample operation tasks sorted based on time; determining the annotation task identifier based on task identifiers of the plurality of sample operation tasks; performing encoding on actions involved in the plurality of sample operation tasks to obtain the annotation action data; and performing encoding on tools used when performing the actions involved in the plurality of sample operation tasks to obtain the annotation tool category.

In some embodiments, the state feedback module is further configured to: determine preparation state data of a next operation task of the current operation task based on the target collaboration state data, the preparation state data being used to represent a situation that the operation subject needs to prepare in advance for the next operation task; and feedback the state of the human-machine collaboration process based on the preparation state data and the personnel state data.

In some embodiments, the state feedback module is further configured to determine a target reminding mode corresponding to the personnel state data and display the preparation state data in the target reminding mode to remind the operation subject to prepare in advance for the next operation task

In some embodiments, the apparatus for feedbacking the human-machine collaboration state based on virtual-real integration further includes a decision control module configured to: perform a decision control based on state data feedback for the human-machine collaboration process, to execute a next operation task of the current operation task in an execution mode matching the next operation task. The execution mode is any one of a human-machine collaboration mode, a human-dominated mode, and a machine-dominated mode.

For a specific description of the apparatus for feedbacking the human-machine collaboration state based on virtual-real integration, the description of the method for feedbacking the human-machine collaboration state based on virtual-real integration above can be a reference, and details are omitted here.

In some embodiments, an electronic device is provided, and an internal structure diagram of the electronic device can be shown in FIG. 12. The electronic device includes a processor, a memory, a communication interface, a display screen, and an input apparatus connected through a system bus. The processor of the electronic device is configured to provide computing capability and control capability. The memory of the electronic device includes a nonvolatile storage medium and an internal memory. The nonvolatile storage medium stores an operating system and a computer program. The internal memory provides an environment for execution of the operating system and the computer program in the nonvolatile storage medium. The communication interface of the electronic device is in communication with an external terminal in a wired or wireless manner. The wireless manner can be realized by WIFI, carrier network, Near Field Communication (NFC), or other technologies. The processor, when executing the computer program, realizes the method for feedbacking the human-machine collaboration state based on virtual-real integration. The display screen of the electronic device may be a liquid crystal display screen or an electronic ink display screen. The input apparatus of the electronic device may be a touch layer covered on the display screen, a button, a trackball, or a touch pad arranged on a shell of the electronic device, or an external keyboard, a touch pad, a mouse, and the like.

Those skilled in the art will appreciate that the structure shown in FIG. 12 is merely a block diagram of a portion of the structure associated with the solution disclosed herein and does not constitute a limitation of the electronic device to which the solution disclosed herein is applied. The electronic device may include more or less components than that shown in the figures, or combine certain components, or have a different arrangement of components.

In some embodiments, a computer device is provided. The computer device includes a memory configured to store a computer program and a processor. The processor, when executing the computer program, implements the method according to any one of the above embodiments.

In some embodiments, a computer-readable storage medium having a computer program stored thereon is provided. A processor, when executing the computer program, performs the method according to any one of the above embodiments.

In some embodiments, a computer program product including instructions is provided. The instructions, when executed by a processor of a computer device, enable the computer device to perform the method according to any one of the above embodiments.

It should be noted that the logics and/or steps represented in the flowchart or described otherwise herein can be, for example, considered as a list of ordered executable instructions for implementing logic functions, and can be embodied in any computer-readable medium that is to be used by or used with an instruction execution system, apparatus, or device (such as a computer-based system, a system including a processer, or any other system that can retrieve and execute instructions from an instruction execution system, apparatus, or device). For the present disclosure, a "computer-readable medium" can be any apparatus that can contain, store, communicate, propagate, or transmit a program to be used by or used with an instruction execution system, apparatus, or device. More specific examples (a non-exhaustive list) of computer-readable mediums include: an electrical connector (electronic device) with one or more wirings, a portable computer disk case (magnetic devices), a Random Access Memory (RAM), a Read Only Memory (ROM), an Erasable Programmable Read Only Memory (EPROM or flash memory), a fiber optic device, and a portable Compact Disk Read Only memory (CDROM). In addition, the computer-readable medium may even be a piece of paper or other suitable medium on which the program can be printed, as the program can be obtained electronically, e.g., by optically scanning the paper or the other medium, and then editing, interpreting, or otherwise processing the scanning result when necessary, and then stored in a computer memory.

## Claims

1. A method for feedbacking a human-machine collaboration state based on virtual-real integration, the method comprising:
acquiring (S110) ergonomic data of an operation subject in a human-machine collaboration process for a current operation task and an operation scene image obtained by shooting the human-machine collaboration process, wherein the operation scene image at least comprises an operation device and a setting parameter of an operation environment;
performing (S120) a human-machine collaboration state recognition based on the operation scene image to obtain target collaboration state data corresponding to the current operation task;
performing (S130) a personnel state recognition based on the ergonomic data to obtain personnel state data of the operation subject; and
feedbacking (S140) a state of the human-machine collaboration process based on the target collaboration state data and the personnel state data;
wherein the ergonomic data comprises eye movement signal data, and the personnel state data comprises task prediction state data; and said performing (S130) the personnel state recognition based on the ergonomic data to obtain the personnel state data of the operation subject comprises:
performing (S210) a gaze position recognition based on the eye movement signal data to obtain a gaze position change of the operation subject, wherein the gaze position change is used to describe a change of a gaze position of the operation subject with an execution time of the current operation task; and
determining (S220) whether the operation subject predicts a next operation task of the current operation task based on the gaze position change to obtain the task prediction state data;
wherein the current operation task corresponds to a current task region range, and the next operation task corresponds to a next task region range; and said determining (S220) whether the operation subject predicts the next operation task of the current operation task based on the gaze position change to obtain the task prediction state data comprises:
determining whether at least part of gaze resources of the operation subject are transferred from the current task region range to the next task region range based on the gaze position change to obtain the task prediction state data.

2. The method according to claim 1, wherein said performing (S210) the gaze position recognition based on the eye movement signal data to obtain the gaze position change of the operation subject comprises:
determining (S310) a gaze point scanning path of the operation subject based on the eye movement signal data; and
determining (S320) the gaze position change of the operation subject based on the gaze point scanning path.

3. The method according to claim 1, wherein:
the ergonomic data comprises at least one of electroencephalogram signal data, electrodermal activity signal data, and heart rate signal data; and
the method further comprises:
performing (S510) a load state recognition based on the electroencephalogram signal data to obtain load state data of the operation subject;
performing (S520) an emotional state recognition based on the electrodermal activity signal data and the heart rate signal data to obtain emotional state data of the operation subject; and
performing (S530) a prewarning for an operating state of the operation subject based on the load state data and/or the emotional state data.

4. The method according to claim 1, wherein said performing (S120) the human-machine collaboration state recognition based on the operation scene image to obtain the target collaboration state data corresponding to the current operation task comprises:
performing the human-machine collaboration state recognition based on the operation scene image to obtain current collaboration state data of the current operation task as the target collaboration state data, or
performing the human-machine collaboration state recognition based on the operation scene image to obtain current collaboration state data of the current operation task, and determining target collaboration state data of an adjacent operation task of the current operation task based on the current collaboration state data.

5. The method according to claim 1, wherein said performing (S120) the human-machine collaboration state recognition based on the operation scene image to obtain the target collaboration state data corresponding to the current operation task comprises:
inputting the operation scene image into a target state recognition model to perform the human-machine collaboration state recognition, to obtain the target collaboration state data.

6. The method according to claim 5, wherein the target state recognition model is obtained by being trained in following operations:
acquiring (S610) a sample scene image obtained by shooting a historical operation process, wherein a label of the sample scene image comprises an annotation task identifier, annotation action data, and an annotation tool category corresponding to the annotation action data;
inputting (S620) the sample scene image into an initial state recognition model for prediction to obtain a prediction task identifier, prediction action data, and a prediction tool category corresponding to the prediction action data; and
updating (S630) the initial state recognition model based on the annotation task identifier, the annotation action data, the annotation tool category, the prediction task identifier, the prediction action data, and the prediction tool category to obtain the target state recognition model.

7. The method according to claim 6, wherein the label of the sample scene image is determined by:
performing (S710) task decomposing on the historical operation process to obtain a plurality of sample operation tasks sorted based on time;
determining (S720) the annotation task identifier based on task identifiers of the plurality of sample operation tasks;
performing encoding on actions involved in the plurality of sample operation tasks to obtain the annotation action data; and
performing (S730) encoding on tools used when performing the actions involved in the plurality of sample operation tasks to obtain the annotation tool category.

8. The method according to any one of claims 1 to 3 and 5 to 7, wherein said feedbacking (S140) the state of the human-machine collaboration process based on the target collaboration state data and the personnel state data comprises:
determining (S810) preparation state data of a next operation task of the current operation task based on the target collaboration state data, wherein the preparation state data is used to represent a situation that the operation subject needs to prepare in advance for the next operation task; and
feedbacking (S820) the state of the human-machine collaboration process based on the preparation state data and the personnel state data.

9. The method according to claim 8, wherein said feedbacking (S820) the state of the human-machine collaboration process based on the preparation state data and the personnel state data comprises:
determining (S910) a target reminding mode corresponding to the personnel state data; and
displaying (S920) the preparation state data in the target reminding mode to remind the operation subject to prepare in advance for the next operation task.

10. The method according to claim 1, further comprising:
performing a decision control based on state data feedback for the human-machine collaboration process, to execute a next operation task of the current operation task in an execution mode matching the next operation task, wherein the execution mode is any one of a human-machine collaboration mode, a human-dominated mode, and a machine-dominated mode.

11. An apparatus for feedbacking a human-machine collaboration state based on virtual-real integration, the apparatus comprising:
a task data acquisition module (1110) configured to acquire ergonomic data of an operation subject in a human-machine collaboration process for a current operation task and an operation scene image obtained by shooting the human-machine collaboration process, wherein the operation scene image at least comprises an operation device and a setting parameter of an operation environment;
a human-machine collaboration state recognition module (1120) configured to perform a human-machine collaboration state recognition based on the operation scene image to obtain target collaboration state data corresponding to the current operation task;
a personnel state recognition module (1130) configured to perform a personnel state recognition based on the ergonomic data to obtain personnel state data of the operation subject; and
a state feedback module (1140) configured to feedback a state of the human-machine collaboration process based on the target collaboration state data and the personnel state data;
wherein the ergonomic data comprises eye movement signal data, and the personnel state data comprises task prediction state data; and the personnel state recognition module (1130) is further configured to:
perform a gaze position recognition based on the eye movement signal data to obtain a gaze position change of the operation subject, wherein the gaze position change is used to describe a change of a gaze position of the operation subject with an execution time of the current operation task; and determine whether the operation subject predicts a next operation task of the current operation task based on the gaze position change to obtain the task prediction state data;
wherein the current operation task corresponds to a current task region range, and the next operation task corresponds to a next task region range; and the personnel state recognition module (1130) is further configured to:
determine whether at least part of gaze resources of the operation subject are transferred from the current task region range to the next task region range based on the gaze position change to obtain the task prediction state data.

12. A computer-readable storage medium having a computer program stored thereon, wherein a processor, when executing the computer program, implements the method according to any one of claims 1 to 10.

## Patentansprüche

1. Verfahren zur Rückmeldung eines Mensch-Maschine-Kollaborationszustandes auf Basis von Virtual-Reality-Integration, wobei das Verfahren umfasst:
Erfassen (S110) ergonomischer Daten eines Bediensubjekts in einem Mensch-Maschine-Kollaborationsprozess für eine aktuelle Arbeitsaufgabe sowie eines durch Aufnahme des Mensch-Maschine-Kollaborationsprozesses gewonnenen Betriebsszenenbildes, wobei das Betriebsszenenbild mindestens eine Betriebsvorrichtung und einen Einstellparameter einer Betriebsumgebung umfasst;
Durchführen (S120) einer Erkennung des Mensch-Maschine-Kollaborationszustands basierend auf dem Betriebsszenenbild, um Ziel-Kollaborationszustandsdaten zu erhalten, die der aktuellen Arbeitsaufgabe entsprechen;
Durchführen (S130) einer Erkennung des Personalzustands basierend auf den ergonomischen Daten, um Personalzustandsdaten des Bediensubjekts zu erhalten; und
Rückmelden (S140) eines Zustands des Mensch-Maschine-Kollaborationsprozesses basierend auf den Ziel-Kollaborationszustandsdaten und den Personalzustandsdaten;
wobei die ergonomischen Daten Augenbewegungssignaldaten umfassen und die Personalzustandsdaten Aufgabenvorhersagezustandsdaten umfassen; und wobei das Durchführen (S130) einer Erkennung der Personalzustands basierend auf den ergonomischen Daten, um die Personalzustandsdaten des Bediensubjekts zu erhalten, umfasst:
Durchführen (S210) einer Erkennung einer Blickposition auf Basis der Augenbewegungssignaldaten, um eine Blickpositionsänderung des Bediensubjekts zu erhalten, wobei die Blickpositionsänderung verwendet wird, um eine Änderung einer Blickposition des Bediensubjekts mit einer Ausführungszeit der aktuellen Arbeitsaufgabe zu beschreiben; und
Bestimmen (S220), ob das Bediensubjekt eine nächste Arbeitsaufgabe der aktuellen Arbeitsaufgabe auf Basis der Blickpositionsänderung vorhersagt, um die Aufgabenvorhersagezustandsdaten zu erhalten;
wobei die aktuelle Arbeitsaufgabe einem aktuellen Aufgabenbereich entspricht und die nächste Arbeitsaufgabe einem nächsten Aufgabenbereich entspricht; und das Bestimmen (S220), ob das Bediensubjekt eine nächste Arbeitsaufgabe der aktuellen Arbeitsaufgabe auf Basis der Blickpositionsänderung vorhersagt, um die Aufgabenvorhersagezustandsdaten zu erhalten;
Bestimmen, ob mindestens ein Teil der Blickressourcen des Bediensubjekts basierend auf der Blickpositionsänderung vom aktuellen Aufgabenbereich zum nächsten Aufgabenbereich übertragen wird, um die Aufgabenvorhersagezustandsdaten zu erhalten.

2. Verfahren nach Anspruch 1, wobei das Durchführen (S210) einer Erkennung einer Blickposition auf Basis der Augenbewegungssignaldaten, um eine Blickpositionsänderung des Bediensubjekts zu erhalten, umfasst:
Bestimmen (S310) eines Blickpunktabtastpfades des Bediensubjekts basierend auf den Augenbewegungssignaldaten; und
Bestimmen (S320) der Blickpositionsänderung des Bediensubjekts auf Basis des Blickpunktabtastpfades.

3. Verfahren nach Anspruch 1, wobei
die ergonomische Date mindestens eines der folgenden: Elektroenzephalogram-Signaldaten, elektrodermale Aktivitätssignaldaten und Herzfrequenz-Signaldaten umfasst; und
wobei das Verfahren ferner umfasst:
Durchführen (S510) einer Erkennung des Belastungszustands basierend auf den Elektroenzephalogramm-Signaldaten, um Belastungszustandsdaten des Bediensubjekts zu erhalten;
Durchführen (S520) einer Erkennung des Emotionszustands basierend auf den elektrodermalen Aktivitätssignaldaten und den Herzfrequenzsignaldaten, um Emotionszustandsdaten des Bediensubjekts zu erhalten; und
Durchführen (S530) einer Vorwarnung für einen Bedienzustand des Bediensubjekts basierend auf den Belastungszustandsdaten und/oder den Emotionszustandsdaten.

4. Verfahren nach Anspruch 1, wobei das Durchführen (S120) einer Erkennung des Mensch-Maschine-Kollaborationszustands basierend auf dem Betriebsszenenbild, um Ziel-Kollaborationszustandsdaten zu erhalten, die der aktuellen Arbeitsaufgabe entsprechen, umfasst:
Durchführen der Erkennung des Mensch-Maschine-Kollaborationszustands basierend auf dem Betriebsszenenbild, um aktuelle Kollaborationszustandsdaten der aktuellen Arbeitsaufgabe als Ziel-Kollaborationszustandsdaten zu erhalten, oder
Durchführen der Erkennung des Mensch-Maschine-Kollaborationszustands basierend auf dem Betriebsszenenbild, um aktuelle Kollaborationszustandsdaten der aktuellen Arbeitsaufgabe zu erhalten, und Bestimmen von Ziel-Kollaborationszustandsdaten einer benachbarten Arbeitsaufgabe der aktuellen Arbeitsaufgabe basierend auf den aktuellen Kollaborationszustandsdaten.

5. Verfahren nach Anspruch 1, wobei das Durchführen (S120) einer Erkennung des Mensch-Maschine-Kollaborationszustands basierend auf dem Betriebsszenenbild, um Ziel-Kollaborationszustandsdaten zu erhalten, die der aktuellen Arbeitsaufgabe entsprechen, umfasst:
Eingeben des Betriebsszenenbildes in ein Zielzustand-Erkennungsmodell zur Durchführung der Erkennung des Mensch-Maschine-Kollaborationszustands, um die Ziel-Kollaborationszustandsdaten zu erhalten.

6. Verfahren nach Anspruch 5, wobei das Zielzustand-Erkennungsmodell durch Training in folgenden Operationen erhalten wird:
Erfassen (S610) eines Beispielszenenbildes, das durch Aufnehmen eines historischen Bediensprozesses erhalten wurde, wobei ein Etikett des Beispielszenenbildes einen Anmerkungsaufgabenkennzeichner, Anmerkungsaktionsdaten und eine Anmerkungswerkzeugkategorie, die den Anmerkungsaktionsdaten entspricht, umfasst;
Eingeben (S620) des Beispielszenenbildes in ein Anfangszustand-Erkennungsmodell zur Vorhersage, um einen Vorhersageaufgabenkennzeichner, Vorhersageaktionsdaten und eine Vorhersagewerkzeugkategorie zu erhalten, die den Vorhersageaktionsdaten entsprechen; und
Aktualisieren (S630) des Anfangszustand-Erkennungsmodells basierend auf dem Anmerkungsaufgabenkennzeichner, den Anmerkungsaktionsdaten, der Anmerkungswerkzeugkategorie, dem Vorhersageaufgabenkennzeichner, den Vorhersageaktionsdaten und der Vorhersagewerkzeugkategorie, um das Zielzustand-Erkennungsmodell zu erhalten.

7. Verfahren nach Anspruch 6, wobei das Etikett des Beispielszenenbilds bestimmt wird durch:
Durchführen (S710) einer Aufgabenzerlegung auf dem historischen Bediensprozess, um eine Vielzahl von Beispielvorgangsaufgaben zu erhalten, die nach der Zeit sortiert sind;
Bestimmen (S720) des Anmerkungsaufgabenkennzeichners auf Basis von Aufgabenkennzeichnern der mehreren Beispielvorgangsaufgaben;
Durchführen der Kodierung auf Aktionen, die an den Beispielvorgangsaufgaben beteiligt sind, um die Anmerkungsaktionsdaten zu erhalten; und
Durchführen (S730) der Kodierung auf Werkzeugen, die beim Durchführen der an den Beispielvorgangsaufgaben beteiligten Aktionen verwendet werden, um die Anmerkungswerkzeugkategorie zu erhalten.

8. Das Verfahren nach einem der Ansprüche 1 bis 3 und 5 bis 7, wobei das Rückmelden (S140) eines Zustands des Mensch-Maschine-Kollaborationsprozesses basierend auf den Ziel-Kollaborationszustandsdaten und den Personalzustandsdaten umfasst:
Bestimmen (S810) von Vorbereitungszustandsdaten einer nächsten Arbeitsaufgabe der aktuellen Arbeitsaufgabe basierend auf der Ziel-Kollaborationszustandsdatei, wobei die Vorbereitungszustandsdaten verwendet werden, um eine Situation darzustellen, in der das Bediensubjekt im Voraus für die nächste Arbeitsaufgabe vorbereiten muss; und
Rückmelden (S820) eines Zustands des Mensch-Maschine-Kollaborationsprozesses basierend auf den Vorbereitungszustandsdaten und den Personalzustandsdaten;

9. Verfahren nach Anspruch 8, wobei das Rückmelden (S820) eines Zustands des Mensch-Maschine-Kollaborationsprozesses basierend auf den Vorbereitungszustandsdaten und den Personalzustandsdaten umfasst:
Bestimmen (S910) eines Zielerinnerungsmodus, der den Personalzustandsdaten entspricht; und
Anzeigen (S920) der Vorbereitungszustandsdaten im Zielerinnerungsmodus, um das Bediensubjekt daran zu erinnern, sich im Voraus auf die nächste Arbeitsaufgabe vorzubereiten.

10. Verfahren nach Anspruch 1, ferner umfassend:
Durchführen einer Entscheidungssteuerung basierend auf Zustandsdatenrückmeldung für den Mensch-Maschine-Kollaborationsprozess, um eine nächste Arbeitsaufgabe der aktuellen Arbeitsaufgabe in einem Ausführungsmodus auszuführen, der zur nächsten Arbeitsaufgabe passt, wobei der Ausführungsmodus einer der folgenden ist: ein Mensch-Maschine-Kollaborationsmodus, ein menschdominierter Modus oder ein maschinendominierter Modus.

11. Vorrichtung zur Rückmeldung eines Mensch-Maschine-Kollaborationszustands auf Basis von Virtual-Reality-Integration, wobei die Vorrichtung umfasst:
ein Aufgabedaten-Erfassungsmodul (1110), das konfiguriert ist, um ergonomische Daten eines Bediensubjekts in einem Mensch-Maschine-Kollaborationsprozess für eine aktuelle Arbeitsaufgabe und ein Betriebsszenenbild zu erfassen, das durch Aufnahme des Mensch-Maschine-Kollaborationsprozesses erhalten wird, wobei das Betriebsszenenbild mindestens eine Betriebsvorrichtung und einen Einstellparameter einer Betriebsumgebung umfasst;
ein Mensch-Maschine-Kollaborationszustand-Erkennungsmodul (1120), das konfiguriert ist, um basierend auf dem Betriebsszenenbild eine Erkennung des Mensch-Maschine-Kollaborationszustands durchzuführen, um die Ziel-Kollaborationszustandsdatei zu erhalten, die der aktuellen Arbeitsaufgabe entspricht;
ein Personalzustand-Erkennungsmodul (1130), das konfiguriert ist, eine Personalzustandserkennung basierend auf den ergonomischen Daten durchzuführen, um Personalzustandsdaten des Bediensubjekts zu erhalten; und
ein Zustandsrückmeldemodul (1140), das konfiguriert ist, um einen Zustand des Mensch-Maschine-Kollaborationsprozesses basierend auf den Ziel-Kollaborationszustandsdaten und den Personalzustandsdaten rückzumelden;
wobei die ergonomischen Daten Augenbewegungssignaldaten umfassen und die Personalzustandsdaten Aufgabenvorhersagezustandsdaten umfassen; und das Personalzustand-Erkennungsmodul (1130) ferner konfiguriert ist, um:
eine Erkennung einer Blickposition auf Basis der Augenbewegungssignaldaten durchzuführen, um eine Blickpositionsänderung des Bediensubjekts zu erhalten, wobei die Blickpositionsänderung verwendet wird, um eine Änderung einer Blickposition des Bediensubjekts mit einer Ausführungszeit der aktuellen Arbeitsaufgabe zu beschreiben; zu bestimmen, ob das Bediensubjekt eine nächste Arbeitsaufgabe der aktuellen Arbeitsaufgabe auf Basis der Blickpositionsänderung vorhersagt, um die Aufgabenvorhersagezustandsdaten zu erhalten;
wobei die aktuelle Arbeitsaufgabe einem aktuellen Aufgabenbereich entspricht und die nächste Arbeitsaufgabe einem nächsten Aufgabenbereich entspricht; und das Personalzustand-Erkennungsmodul (1130) ferner konfiguriert ist, um:
zu bestimmen, ob mindestens ein Teil der Blickressourcen des Bediensubjekts basierend auf der Blickpositionsänderung vom aktuellen Aufgabenbereich zum nächsten Aufgabenbereich übertragen wird, um die Aufgabenvorhersagezustandsdaten zu erhalten.

12. Computerlesbares Speichermedium mit einem darauf gespeicherten Computerprogramm, wobei ein Prozessor bei Ausführung des Computerprogramms das Verfahren nach einem der Ansprüche 1 bis 10 implementiert.

## Revendications

1. Procédé de retour d'état de collaboration homme-machine basé sur l'intégration virtuelle-réelle, le procédé comprenant :
l'acquisition (S110) de données ergonomiques d'un sujet opérateur lors du processus de collaboration homme-machine pour une tâche opérationnelle actuelle, ainsi que d'une image de scène opérationnelle obtenue par capture du processus de collaboration homme-machine, ladite image de scène opérationnelle comprenant au moins un équipement opérationnel et un paramètre de réglage d'un environnement opérationnel ;
la reconnaissance (S120) de l'état de collaboration homme-machine à partir de l'image de scène opérationnelle pour obtenir des données cibles d'état de collaboration correspondant à la tâche opérationnelle actuelle ;
la reconnaissance (S130) de l'état du personnel à partir des données ergonomiques pour obtenir des données d'état du personnel du sujet opérateur ; et
le retour (S140) de l'état du processus de collaboration homme-machine à partir des données cibles d'état de collaboration et des données d'état du personnel ;
où les données ergonomiques comprennent des données de signal oculomoteur, et les données d'état du personnel comprennent des données d'état de prédiction de tâche ; et la reconnaissance (S130) de l'état du personnel à partir des données ergonomiques pour obtenir les données d'état du personnel du sujet opérateur comprend :
la reconnaissance (S210) de la position de regard à partir des données de signal oculomoteur pour obtenir un changement de position de regard du sujet opérateur, ledit changement de position de regard étant utilisé pour décrire l'évolution de la position de regard du sujet opérateur en fonction du temps d'exécution de la tâche opérationnelle actuelle ; et
la détermination (S220) de savoir si le sujet opérateur prédit une tâche opérationnelle suivante de la tâche opérationnelle actuelle à partir du changement de position de regard pour obtenir les données d'état de prédiction de tâche ;
où la tâche opérationnelle actuelle correspond à une plage de région de tâche actuelle, et la tâche opérationnelle suivante correspond à une plage de région de tâche suivante ; et la détermination (S220) de savoir si le sujet opérateur prédit la tâche opérationnelle suivante de la tâche opérationnelle actuelle à partir du changement de position de regard pour obtenir les données d'état de prédiction de tâche comprend :
la détermination de savoir si au moins une partie des ressources de regard du sujet opérateur est transférée de la plage de région de tâche actuelle à la plage de région de tâche suivante à partir du changement de position de regard pour obtenir les données d'état de prédiction de tâche.

2. Procédé selon la revendication 1, où ladite reconnaissance (S210) de la position de regard à partir des données de signal oculomoteur pour obtenir un changement de position de regard du sujet opérateur comprend :
la détermination (S310) d'un trajet de balayage du point de regard du sujet opérateur à partir des données de signal oculomoteur ; et
la détermination (S320) du changement de position de regard du sujet opérateur à partir du trajet de balayage du point de regard.

3. Procédé selon la revendication 1, où :
les données ergonomiques comprennent au moins l'une parmi des données de signal électroencéphalographique, des données de signal d'activité électrodermale et des données de signal de fréquence cardiaque ; et
le procédé comprend en outre :
la reconnaissance (S510) de l'état de charge à partir des données de signal électroencéphalographique pour obtenir des données d'état de charge du sujet opérateur ;
la reconnaissance (S520) de l'état émotionnel à partir des données de signal d'activité électrodermale et des données de signal de fréquence cardiaque pour obtenir des données d'état émotionnel du sujet opérateur ; et
l'alerte (S530) relatives à l'état opérationnel du sujet opérateur à partir des données d'état de charge et/ou des données d'état émotionnel.

4. Procédé selon la revendication 1, où ladite reconnaissance (S120) de l'état de collaboration homme-machine à partir de l'image de scène opérationnelle pour obtenir des données cibles d'état de collaboration correspondant à la tâche opérationnelle actuelle comprend :
la reconnaissance de l'état de collaboration homme-machine à partir de l'image de scène opérationnelle pour obtenir des données actuelles d'état de collaboration de la tâche opérationnelle actuelle en tant que données cibles d'état de collaboration, ou
la reconnaissance de l'état de collaboration homme-machine à partir de l'image de scène opérationnelle pour obtenir des données actuelles d'état de collaboration de la tâche opérationnelle actuelle, et la détermination de données cibles d'état de collaboration d'une tâche opérationnelle adjacente à la tâche opérationnelle actuelle à partir des données actuelles d'état de collaboration.

5. Procédé selon la revendication 1, où ladite reconnaissance (S120) de l'état de collaboration homme-machine à partir de l'image de scène opérationnelle pour obtenir des données cibles d'état de collaboration correspondant à la tâche opérationnelle actuelle comprend :
l'entrée de l'image de scène opérationnelle dans un modèle de reconnaissance d'état cible pour effectuer la reconnaissance de l'état de collaboration homme-machine, afin d'obtenir les données cibles d'état de collaboration.

6. Procédé selon la revendication 5, où le modèle de reconnaissance d'état cible est obtenu par entraînement selon les opérations suivantes :
l'acquisition (S610) d'une image de scène échantillon obtenue par capture d'un processus opérationnel historique, où une étiquette de l'image de scène échantillon comprend un identifiant de tâche annoté, des données d'action annotées et une catégorie d'outil annotée correspondant aux données d'action annotées ;
l'entrée (S620) de l'image de scène échantillon dans un modèle initial de reconnaissance d'état pour effectuer une prédiction, afin d'obtenir un identifiant de tâche prédit, des données d'action prédites et une catégorie d'outil prédite correspondant aux données d'action prédites ; et
la mise à jour (S630) du modèle initial de reconnaissance d'état à partir de l'identifiant de tâche annoté, des données d'action annotées, de la catégorie d'outil annotée, de l'identifiant de tâche prédit, des données d'action prédites et de la catégorie d'outil prédite pour obtenir le modèle de reconnaissance d'état cible.

7. Procédé selon la revendication 6, où l'étiquette de l'image de scène échantillon est déterminée par :
la décomposition (S710) de tâche du processus opérationnel historique pour obtenir une pluralité de tâches opérationnelles échantillon triées selon le temps ;
la détermination (S720) de l'identifiant de tâche annoté à partir des identifiants de tâche de la pluralité de tâches opérationnelles échantillon ;
l'encodage des actions impliquées dans la pluralité de tâches opérationnelles échantillon pour obtenir les données d'action annotées ; et
l'encodage (S730) des outils utilisés lors de l'exécution des actions impliquées dans la pluralité de tâches opérationnelles échantillon pour obtenir la catégorie d'outil annotée.

8. Procédé selon l'une quelconque des revendications 1 à 3 et 5 à 7, où ledit retour (S140) de l'état du processus de collaboration homme-machine à partir des données cibles d'état de collaboration et des données d'état du personnel comprend :
la détermination (S810) de données d'état de préparation d'une tâche opérationnelle suivante de la tâche opérationnelle actuelle à partir des données cibles d'état de collaboration, lesdites données d'état de préparation étant utilisées pour représenter la situation de préparation que le sujet opérateur doit effectuer à l'avance pour la tâche opérationnelle suivante ; et
le retour (S820) de l'état du processus de collaboration homme-machine à partir des données d'état de préparation et des données d'état du personnel.

9. Procédé selon la revendication 8, où ledit retour (S820) de l'état du processus de collaboration homme-machine à partir des données d'état de préparation et des données d'état du personnel comprend :
la détermination (S910) d'un mode de rappel cible correspondant aux données d'état du personnel ; et
l'écran d'affichage (S920) des données d'état de préparation selon le mode de rappel cible pour rappeler au sujet opérateur de préparer à l'avance la tâche opérationnelle suivante.

10. Procédé selon la revendication 1, comprenant en outre :
l'exécution d'une commande de décision à partir du retour de données d'état du processus de collaboration homme-machine, afin d'exécuter une tâche opérationnelle suivante de la tâche opérationnelle actuelle selon un mode d'exécution adapté à la tâche opérationnelle suivante, ledit mode d'exécution étant l'un parmi un mode de collaboration homme-machine, un mode à dominance humaine et un mode à dominance machine.

11. Appareil de retour d'état de collaboration homme-machine basé sur l'intégration virtuelle-réelle, l'appareil comprenant :
un module d'acquisition de données de tâche (1110) configuré pour acquérir des données ergonomiques d'un sujet opérateur lors du processus de collaboration homme-machine pour une tâche opérationnelle actuelle, ainsi que d'une image de scène opérationnelle obtenue par capture du processus de collaboration homme-machine, ladite image de scène opérationnelle comprenant au moins un équipement opérationnel et un paramètre de réglage d'un environnement opérationnel ;
un module de reconnaissance d'état de collaboration homme-machine (1120) configuré pour effectuer une reconnaissance de l'état de collaboration homme-machine à partir de l'image de scène opérationnelle pour obtenir des données cibles d'état de collaboration correspondant à la tâche opérationnelle actuelle ;
un module de reconnaissance d'état du personnel (1130) configuré pour effectuer une reconnaissance de l'état du personnel à partir des données ergonomiques pour obtenir des données d'état du personnel du sujet opérateur ; et
un module de retour d'état (1140) configuré pour effectuer un retour de l'état du processus de collaboration homme-machine à partir des données cibles d'état de collaboration et des données d'état du personnel ;
où les données ergonomiques comprennent des données de signal oculomoteur, et les données d'état du personnel comprennent des données d'état de prédiction de tâche ; et le module de reconnaissance d'état du personnel (1130) est en outre configuré pour :
effectuer une reconnaissance de la position de regard à partir des données de signal oculomoteur pour obtenir un changement de position de regard du sujet opérateur, ledit changement de position de regard étant utilisé pour décrire l'évolution de la position de regard du sujet opérateur en fonction du temps d'exécution de la tâche opérationnelle actuelle ; et déterminer si le sujet opérateur prédit une tâche opérationnelle suivante de la tâche opérationnelle actuelle à partir du changement de position de regard pour obtenir les données d'état de prédiction de tâche ;
où la tâche opérationnelle actuelle correspond à une plage de région de tâche actuelle, et la tâche opérationnelle suivante correspond à une plage de région de tâche suivante ; et le module de rec onnaissance d'état du personnel (1130) est en outre configuré pour :
déterminer si au moins une partie des ressources de regard du sujet opérateur est transférée de la plage de région de tâche actuelle à la plage de région de tâche suivante à partir du changement de position de regard pour obtenir les données d'état de prédiction de tâche.

12. Support de stockage lisible par ordinateur sur lequel est stocké un programme informatique, où un processeur, lors de l'exécution du programme informatique, met en œuvre le procédé selon l'une quelconque des revendications 1 à 10.
